# EUROPEAN PATENT APPLICATION

(11) **EP 1 905 759 A1**
(43) Date of publication of application: **02.04.2008**
(21) Application number: 06018795.2
(22) Date of filing: 07.09.2006
(51) Int. Cl.: C07D 207/12, A61K 31/40, A61P 3/10

(54) **N-[1-(3-AMINO-4-PHENYL-BUTYRYL)-4-HYDROXY-PYRROLIDIN-2-YLMETHYL}-PROPIONAMIDE AND RELATED COMPOUNDS AS DPP-IV INHIBITORS FOR THE TREATMENT OFTYPE 2 DIABETES MELLITUS**

(71) Applicant: Santhera Pharmaceuticals (Schweiz) AG, 4410 Liestal (CH); BIOVITRUM AB, 112 76 Stockholm (SE)
(72) Inventor: BULAT, Stephan, 79541 Lörach (DE); EDKUND, Per Olof, 11352 Stockholm (SE)
(74) Representative: Grünecker, Kinkeldey, Stockmair & Schwanhäusser Anwaltssozietät

(57) **Abstract**

The invention relates to compounds of formula (I) and formula (II) 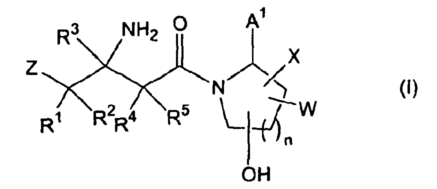 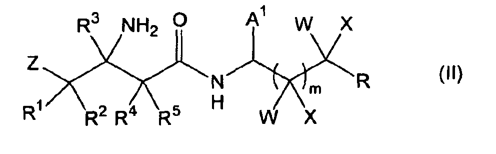 wherein Z, R¹⁻⁵, R, W, X, Y, m, n and A¹ have the meaning as cited in the description and the claims. Said compounds are useful as DPP-IV inhibitors. The invention also relates to the preparation of such compounds as well as the production and use thereof as medicament.

## Description

The present invention relates to a novel class of dipeptidyl peptidase inhibitors, including pharmaceutically acceptable salts and prodrugs thereof, which are useful as therapeutic compounds, particularly in the treatment of Type 2 diabetes mellitus, often referred to as non-insulin dependent diabetes mellitus (NIDDM), and of conditions that are often associated with this disease, such as obesity and lipid disorders.

Diabetes refers to a disease process derived from multiple causative factors and characterized by elevated levels of plasma glucose or hyperglycemia in the fasting state or after administration of glucose during an oral glucose tolerance test. Persistent or uncontrolled hyperglycemia is associated with increased and premature morbidity and mortality. Often abnormal glucose homeostasis is associated both directly and indirectly with alterations of the lipid, lipoprotein and apolipoprotein metabolism and other metabolic and hemodynamic disease. Therefore patients with Type 2 diabetes mellitus are at an increased risk of macrovascular and microvascular complications, including coronary heart disease, stroke, peripheral vascular disease, hypertension, nephropathy, neuropathy, and retinopathy. Therefore, therapeutical control of glucose homeostasis, lipid metabolism and hypertension are critically important in the clinical management and treatment of diabetes mellitus.

There are two generally recognized forms of diabetes. In Type 1, or insulin-dependent, diabetes mellitus (IDDM), patients produce little or no insulin, which is the hormone regulating glucose utilization. In Type 2, or noninsulin dependent, diabetes mellitus (NIDDM), patients often have plasma insulin levels that are the same or elevated compared to nondiabetic subjects. These patients develop a resistance to the insulin stimulating effect on glucose and lipid metabolism in the main insulin-sensitive tissues, namely the muscle, liver and adipose tissues. Further, the plasma insulin levels, while elevated, are insufficient to overcome the pronounced insulin resistance.

Insulin resistance is not primarily due to a diminished number of insulin receptors but to a post-insulin receptor binding defect that is not yet understood. This resistance to insulin responsiveness results in insufficient insulin activation of glucose uptake, oxidation and storage in muscle, and inadequate insulin repression of lipolysis in adipose tissue and of glucose production and secretion in the liver.

The available treatments for Type 2 diabetes, which have not changed substantially in many years, have recognized limitations. While physical exercise and reductions in dietary intake of calories will dramatically improve the diabetic condition, compliance with this treatment is very poor because of well-entrenched sedentary lifestyles and excess food consumption, especially of foods containing high amounts of saturated fat. Increasing the plasma level of insulin by administration of sulfonylureas (e.g., tolbutamide and glipizide) or meglitinide, which stimulate the pancreatic β-cells to secrete more insulin, and/or by injection of insulin when sulfonylureas or meglitinide become ineffective, can result in insulin concentrations high enough to stimulate the very insulin-resistant tissues. However, dangerously low levels of plasma glucose can result from administration of insulin or insulin secretagogues (sulfonylureas or meglitinide), and an increased level of insulin resistance, due to the even higher plasma insulin levels, can occur. The biguanides increase insulin sensitivity resulting in some correction of hyperglycemia. However, the two biguanides, phenformin and metformin, can induce lactic acidosis and nausea/diarrhoea. Metformin has fewer side effects than phenformin and is often prescribed for the treatment of Type 2 diabetes.

The glitazones (*i.e.,* 5-benzylthiazolidine-2,4-diones) are a recently described class of compounds with potential for ameliorating many symptoms of Type 2 diabetes. These agents substantially increase insulin sensitivity in muscle, liver and adipose tissue in several animal models of Type 2 diabetes, resulting in partial or complete correction of the elevated plasma levels of glucose without occurrence of hypoglycemia. The glitazones that are currently marketed are agonists of the peroxisome proliferator activated receptor (PPAR), primarily the PPAR-gamma subtype. PPAR-gamma agonism is generally believed to be responsible for the improved insulin sensitization that is observed with the glitazones. Newer PPAR agonists that are being tested for treatment of Type 2 diabetes are agonists of the alpha, gamma or delta subtype, or a combination of these, and in many cases are chemically different from the glitazones (*i.e*., they are not thiazolidinediones). Serious side effects (*e.g*., liver toxicity) have occurred with some of the glitazones, such as troglitazone.

Additional methods of treating the disease are still under investigation. New biochemical approaches that have been recently introduced or are still under development include treatment with alpha-glucosidase inhibitors (*e.g*., acarbose) and protein tyrosine phosphatase-IB (PTP-1 B) inhibitors.

Compounds that are inhibitors of the dipeptidyl peptidase-IV (DPP-IV) enzyme are also under investigation as drugs that may be useful in the treatment of diabetes, and particularly Type 2 diabetes; see for example WO-A-97/40832, WO-A-98/19998, WO-A-03/00180 and WO-A-03/00181. The usefulness of DPP-IV inhibitors in the treatment of Type 2 diabetes is based on the fact that DPP-IV *in vivo* readily inactivates glucagon like peptide-1 (GLP-1) and gastric inhibitory peptide (GIP). GLP-1 and GIP are incretins and are produced when food is consumed. The incretins stimulate production of insulin. Inhibition of DPP-IV leads to decreased inactivation of the incretins, and this in turn results in increased effectiveness of the incretins in stimulating production of insulin by the pancreas. DPP-IV inhibition therefore results in an increased level of serum insulin. Advantageously, since the incretins are produced by the body only when food is consumed, DPP-IV inhibition is not expected to increase the level of insulin at inappropriate times, such as between meals, which can lead to excessively low blood sugar (hypoglycemia). Inhibition of DPP-IV is therefore expected to increase insulin without increasing the risk of hypoglycemia, which is a dangerous side effect associated with the use of insulin secretagogues.

DPP-IV inhibitors may also have other therapeutic utilities, as discussed elsewhere in this application. DPP-IV inhibitors have not been studied extensively to date, especially for utilities other than diabetes. New compounds are needed so that improved DPP-IV inhibitors can be found for the treatment of diabetes and potentially other disease and conditions.

Thus, the object of the present invention is to provide a new class of DPP-IV inhibitors which is effective in the treatment of Type 2 diabetes and other DPP-IV modulated diseases.

Accordingly, the present invention provides novel compounds of formula (I) and formula (II) as defined in claims 1 to 26.

Preferably, the present invention provides novel compounds of formula (I): 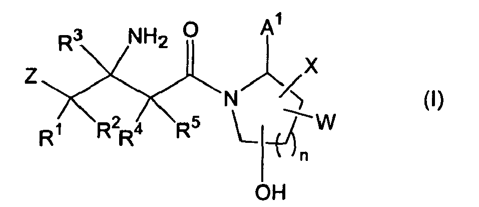 or a pharmaceutically acceptable salt thereof, wherein
Z is selected from the group consisting of phenyl;
naphthyl;
C₃₋₇ cycloalkyl;
heterocycle; and
heterobicycle;
   wherein Z is optionally substituted with one, or independently from each other, more of
   halogen;
   CN;
   OH;
   =O, where the ring is at least partially saturated;
   C₁₋₆ alkyl, optionally substituted with one or more F; and
   O-C₁₋₆ alkyl, optionally substituted with one or more F;
R¹, R², R⁴, and R⁵ are independently from each other selected from the group consisting of
H;
F;
OH;
C₁₋₆ alkyl, optionally substituted with one or more F; and
O-C₁₋₆ alkyl, optionally substituted with one or more F;
and/or R¹ and R² optionally form together C₃₋₇ cycloalkyl, which is optionally substituted with one or more F;
and/or R² and R³ optionally form together C₃₋₇ cycloalkyl, which is optionally substituted with one or more F;
and/or R³ and R⁴ optionally form together C₃₋₇ cycloalkyl, which is optionally substituted with one or more F;
and/or R⁴ and R⁵ optionally form together C₃₋₇ cycloalkyl, which is optionally substituted with one or more F;
R³ is H or C₁₋₆ alkyl;
n is 0, 1 or 2;
X is
H;
F;
CH₃;
OCH₃;
OH;
W is
H;
F;
A¹ is
-C(R⁶R⁷)-Y-T;
R⁶ and R⁷ are independently from each other selected from the group consisting of H;
F; and
C₁₋₆ alkyl, optionally substituted with one or more F;
or R⁶ and R⁷ optionally form together C₃₋₇ cycloalkyl, which is optionally substituted with one or more F;
Y is selected from the group consisting of
-O-;
-C₁₋₆ alkyl-O-;
-N(R⁸)-;
-C₁₋₆ alkyl-N(R⁸)-;
-S-;
-C₁₋₆ alkyl-S-;
-S(O)-;
-C₁₋₆ alkyl-S(O)-;
-S(O)₂-; and
-C₁₋₆ alkyl-S(O)₂-;
   wherein each C₁₋₆ alkyl is optionally substituted with one or more F;
R⁸ and T are independently from each other T¹-T² or T²;
T¹ is selected from the group consisting of
-C₁₋₆ alkyl-;
-C₁₋₆ alkyl-O-;
-C₁₋₆ alkyl-N(R⁹)-;
-C(O)-;
-C(O)-C₁₋₆ alkyl-;
-C(O)-C₁₋₆ alkyl-O-;
-C(O)-C₁₋₆ alkyl-N(R⁹)-;
-C(O)O-;
-C(O)O-C₁₋₆ alkyl-;
-C(O)O-C₁₋₆ alkyl-O-;
-C(O)O-C₁₋₆ alkyl-N(R⁹)-;
-C(O)N(R⁹)-;
-C(O)N(R⁹)-C₁₋₆ alkyl-;
-C(O)N(R⁹)-C₁₋₆ alkyl-O-;
-C(O)N(R⁹)-C₁₋₆ alkyl-N(R¹⁰)-;
-S(O)₂-;
-S(O)₂-C₁₋₆ alkyl-;
-S(O)₂-C₁₋₆ alkyl-O-; and
-S(O)₂-C₁₋₆ alkyl-N(R⁹)-;
   wherein each C₁₋₆ alkyl is optionally substituted with one or more F;
R⁹ and R¹⁰ are independently from each other H or C₁₋₆ alkyl, optionally substituted with one or more F;
T² is selected from the group consisting of
H;
F;
CF₃;
C₁₋₆ alkyl, optionally substituted with one or more halogen atoms;
phenyl;
naphthyl;
   wherein phenyl and naphthyl are optionally substituted with one, or independently from each other, more of
   halogen;
   CN;
   R¹¹;
   COOH;
   OH;
   C(O)NH₂;
   S(O)₂NH₂;
   COOT³;
   OT³;
   C(O)NHT³;
   S(O)₂NHT³; or
   T³;
C₃₋₇ cycloalkyl;
heterocycle; and
heterobicycle;
   wherein C₃₋₇ cycloalkyl, heterocycle and heterobicycle are optionally substituted with one, or independently from each other, more of
   halogen;
   CN;
   R¹²;
   OH;
   =O, where the ring is at least partially saturated;
   NH₂;
   COOH;
   C(O)NH₂;
   S(O)₂NH₂;
   COOT³;
   OT³;
   C(O)NHT³;
   S(O)₂NHT³;
   NHT³; or
   T³;
   whereby when R⁸ is T¹-T² and represents -C₁₋₆ alkyl and T is T¹-T² and represents - C₁₋₆ alkyl then R⁸ and T may form together a 3 to 7 membered cyclic group containing one nitrogen atom;
R¹¹ is selected from the group consisting of
C₁₋₆ alkyl;
O-C₁₋₆ alkyl;
COO-C₁₋₆ alkyl;
OC(O)-C₁₋₆ alkyl;
C(O)N(R¹⁴)-C₁₋₆ alkyl;
S(O)₂N(R¹⁶)-C₁₋₆ alkyl;
S(O)-C₁₋₆ alkyl;
S(O)₂-C₁₋₆ alkyl; and
N(R¹⁷)S(O)₂-C₁₋₆ alkyl;
   wherein each C₁₋₆ alkyl is optionally substituted with one, or independently from each other, more of F, COOR¹⁸, C(O)N(R¹⁹R²⁰), S(O)₂N(R²¹R²²), OR²³, N(R²⁴R²⁵), T³, O-T³ or N(R²⁸)-T³;
R¹² is selected from the group consisting of
C₁₋₆ alkyl;
O-C₁₋₆ alkyl;
N(R¹³)-C₁₋₆ alkyl;
COO-C₁₋₆ alkyl;
OC(O)-C₁₋₆ alkyl;
C(O)N(R¹⁴)-C₁₋₆ alkyl;
N(R¹⁵)-C(O)-C₁₋₆ alkyl;
S(O)₂N(R¹⁶)-C₁₋₆ alkyl;
S(O)-C₁₋₆ alkyl;
S(O)₂-C₁₋₆ alkyl; and
-N(R¹⁷)S(O)₂-C₁₋₆ alkyl;
   wherein each C₁₋₆ alkyl is optionally substituted with one, or independently from each other, more of F, COOR¹⁸, C(O)N(R¹⁹R²⁰), S(O)₂N(R²¹R²²), OR²³, N(R²⁴R²⁵), T³, O-T³ or N(R²⁶)-T³;
R¹³, R¹⁴, R¹⁵, R¹⁶, R¹⁷, R¹⁸, R¹⁹, R²⁰, R²¹, R²², R²³, R²⁴, R²⁵, R²⁶, are independently from each other H or C₁₋₆ alkyl;
T³ is selected from the group consisting of phenyl;
naphthyl;
   wherein phenyl and naphthyl are optionally substituted with one, or independently from each other, more of
   halogen;
   CN;
   COOH;
   OH;
   C(O)NH₂;
   S(O)₂NH₂;
   C₁₋₆ alkyl;
   O-C₁₋₆ alkyl;
   COO-C₁₋₆ alkyl;
   OC(O)-C₁₋₆ alkyl;
   C(O)N(R²⁷)-C₁₋₆ alkyl;
   S(O)₂N(R²⁸)-C₁₋₆ alkyl;
   S(O)₂-C₁₋₆ alkyl; or
   N(R²⁹)S(O)₂-C₁₋₆ alkyl;
heterocycle;
heterobicycle; and
C₃₋₇ cycloalkyl;
   wherein the C₃₋₇ cycloalkyl, heterocycle and heterobicycle are optionally substituted with one, or independently from each other, more of
   halogen;
   CN;
   OH;
   =O, where the ring is at least partially saturated;
   NH₂;
   COOH;
   C(O)NH₂;
   S(O)₂NH₂;
   C₁₋₆ alkyl;
   O-C₁₋₆ alkyl;
   N(R³⁰)-C₁₋₆ alkyl;
   COO-C₁₋₆ alkyl;
   OC(O)-C₁₋₆ alkyl;
   C(O)N(R³¹)-C₁₋₆ alkyl;
   N(R³²)-C(O)-C₁₋₆ alkyl;
   S(O)₂N(R³³)-C₁₋₆ alkyl;
   S(O)₂-C₁₋₆ alkyl; or
   -N(R³⁴)S(O)₂-C₁₋₆ alkyl;
   and
R²⁷, R²⁸, R²⁹, R³⁰, R³¹, R³², R³³ and R³⁴ are independently from each other H or C₁₋₆ alkyl.

A preferred stereochemistry of compounds according to the present invention is shown in formula (Ia)

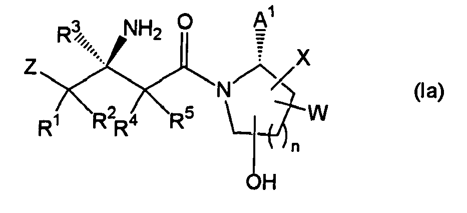

Preferred compounds of formula (I) or (la) are those compounds in which one or more of the residues contained therein have the meanings given below. It is understood, that the claimed compounds cover any compound obtained by combining any of the definitions disclosed within this description for the various substituents. With respect to all compounds of the formulas (I) or (la) the present invention also includes all tautomeric and stereoisomeric forms and mixtures thereof in all ratios, and their pharmaceutically acceptable salts.

In preferred embodiments of the present invention, the substituents R¹ - R⁵, Z, X, W, n and A¹ of the formula (I) or (Ia) independently from each other have the following meaning. Hence, one or more of the substituents R¹ - R⁵, Z, X, W, n and A¹ can have the preferred or more preferred meanings given below.

Z is preferably phenyl or heterocycle and Z is optionally substituted independently from each other with 1, 2 or 3, more preferably with 2 or 3 substituents selected from CI, F, CN, CH₃ or OCH₃. In one embodiment Z is substituted with 1, 2 or 3 F.

It is preferred that R¹, R², R⁴ and R⁵ are independently from each other selected from the group consisting of H, F, OH, CH₃ and OCH₃.

R³ is preferably H.

X is preferably H or F.

W is preferably H. In an other embodiment, W and X both represent F. In a preferred embodiment, W and X both represent F and are both attached to the same carbon atom.

Preferably, n is 1. In other embodiments, n is 0 or 2.

Y is preferably -O-, -N(R⁸) or -S(O)₂-, more preferably -O- or -N(R⁸)-. Most preferably, Y is -N(R⁸)-.

Preferably, R⁸ is selected from the group consisting of H, CH₃, COOH, COOCH₃, C(O)NH₂, C(O)N(CH₃)₂, and S(O)₂CH₃, more preferably H, CH₃, most preferably H.

It is preferred that T is T¹-T² or T², wherein T¹ is selected from the group consisting of
-CH₂-;
-C(O)-;
-C(O)-CH₂-;
-C(O)O-;
-C(O)O-CH₂-;
-C(O)NH-;
-C(O)NH-CH₂-;
-S(O)₂-; and
-S(O)₂-CH₂-.

More preferred is T¹ selected from the group consisting of -C(O)-; -CH₂-; -S(O)₂-; and -C(O)NH-.

It is preferred that T is T¹-T². In this case, T¹-T² is preferably a group as defined below.

In one embodiment, T¹-T² is preferably CH₂-phenyl, whereby phenyl may be substituted with 1 to 3, preferably 1 or 2, substituents selected from halogen, CN, O-C₁₋₄ alkyl, C₁₋₄ alkyl or S(O)₂CH₃, preferably F, CI, O-Me, Me or S(O)₂CH₃.
In one embodiment, T¹-T² is preferably CH₂-C₃₋₇ cycloalkyl, more preferably cyclopropyl or cyclobutyl, more preferably cyclopropyl, whereby cycloalkyl may be substituted with 1 or 2, preferably 1, substituents selected from halogen, CN, OH, NH₂, COOH, C(O)NH₂ or S(O)₂NH₂, more preferably COOH or C(O)NH₂.
In one embodiment, T¹-T² is preferably C₁₋₄ alkyl, preferably methyl, ethyl or propyl, most preferably methyl.
In one embodiment, T¹-T² is preferably C(O)-phenyl, whereby phenyl may be substituted with 1-3, preferably 1 or 2, substituents selected from halogen, CN, O-C₁₋₄alkyl, C₁₋₄ alkyl or S(O)₂CH₃, preferably F, Cl, O-Me, Me or S(O)₂CH₃.
In one embodiment, T¹-T² is preferably C(O)-C₃₋₇ cycloalkyl, more preferably cyclopropyl or cyclobutyl, more preferably cyclopropyl, whereby cycloalkyl may be substituted with 1 to 3, preferably 1 or 2, substituents selected from halogen, CN, O-C₁₋₄ alkyl and C₁₋₄ alkyl, whereby alkyl may be further substituted with 1 to 3 F. More preferably, cycloalkyl may be substituted with one C₁₋₄ alkyl substituted with 1 to 3 F.
In one embodiment, T¹-T² is preferably C(O)-heterocycle, whereby heterocycle may be substituted with 1-3, preferably 1 or 2, substituents selected from halogen, CN, O-C₁₋₄ alkyl, C₁₋₄ alkyl or S(O)₂CH₃; preferably, the heterocycle is aromatic, more preferably containing 1 or 2 heteroatoms selected from N and O, most preferably N. In one embodiment, T¹-T² is preferably S(O)₂-phenyl, whereby phenyl may be substituted with 1-3, preferably 1 or 2, substituents selected from halogen, CN, O-C₁₋₄ alkyl, C₁₋₄ alkyl or S(O)₂CH₃, preferably F, Cl, O-Me, Me or S(O)₂CH₃.
In one embodiment, T¹-T² is preferably S(O)₂-C₃₋₇ cycloalkyl, more preferably cyclopropyl or cyclobutyl, more preferably cyclopropyl, whereby cycloalkyl may be substituted with 1-3, preferably 1 or 2, substituents selected from halogen, CN, O-C₁₋₄ alkyl, C₁₋₄ alkyl, whereby alkyl may be further substituted with 1 to 3 F; more preferably cycloalkyl may be substituted with one C₁₋₄ alkyl substituted with 1 to 3 F. In one embodiment, T¹-T² is preferably S(O)₂-C₁₋₄ alkyl, preferably S(O)₂CH₃.
In one embodiment, T¹-T² is preferably C(O)-NH-phenyl, whereby phenyl may be substituted with 1-3, preferably 1 or 2, substituents selected from halogen, CN, O-C₁₋₄ alkyl, C₁₋₄ alkyl or S(O)₂CH₃.

When T is T², T² is preferably a group as defined below.

In one embodiment, T² is preferably H.
In a further embodiment, T² is preferably phenyl, whereby phenyl may be substituted with 1-3, preferably 1 or 2, substituents selected from halogen, CN, O-C₁₋₄ alkyl, C₁₋₄ alkyl or S(O)₂CH₃, preferably F, Cl, O-Me, Me or S(O)₂CH₃.
In one embodiment, T² is preferably heterocycle, whereby heterocycle may be substituted with 1-3, preferably 1 or 2, substituents selected from halogen, CN, phenyl, heterocycle, O-C₁₋₄ alkyl, C₁₋₄ alkyl or S(O)₂CH₃; preferably, the heterocycle is aromatic, more preferably containing 1, 2 or 3 heteroatoms selected from N and O, most preferably N. When the heterocycle is substituted with phenyl or heterocycle, the heterocycle is preferably aromatic, more preferably containing 1, 2 or 3 heteroatoms selected from N and O, most preferably N, and the phenyl or heterocycle may be further substituted by 1 or 2 F or S(O)₂CH₃.

In one embodiment, T² is preferably CF₃.

More preferably, T² is phenyl or heterocycle.

Preferably, A¹ is -CH₂-N(R⁸)-T, wherein R⁸ is H, S(O)₂CH₃ or S(O)₂-C₃₋₇ cycloalkyl, most preferably H.

In other embodiments, A¹ is -CH₂-O-T.

In the case that Y contains the group R⁸, the following is preferred:
When R⁸ is T¹-T² and represents -C₁₋₆ alkyl and T is T¹-T² and represents -C₁₋₆ alkyl then R⁸ and T may form together a 3 to 7 membered cyclic group containing one nitrogen atom, preferably a 5 or 6 membered cyclic group.

Preferred embodiments of the compounds according to present invention are shown below.

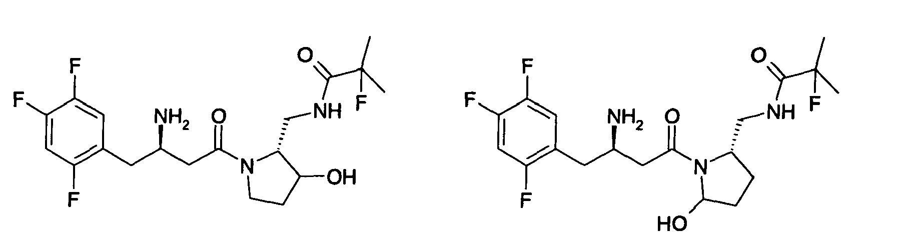

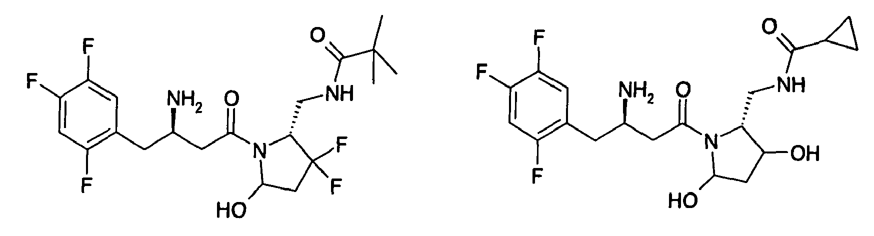

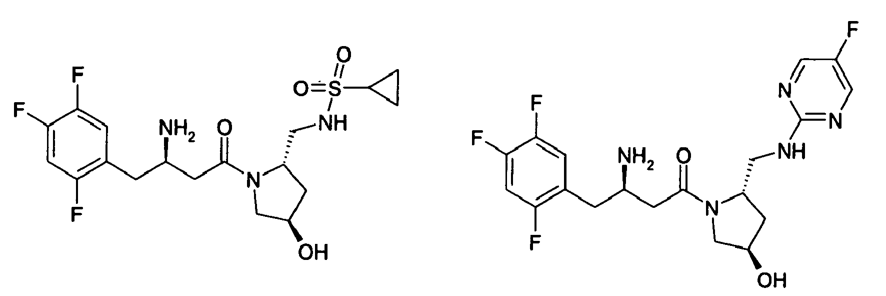

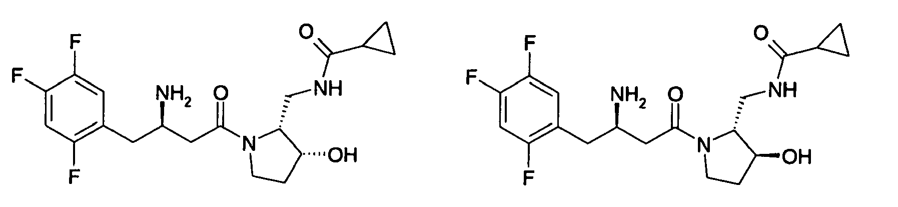

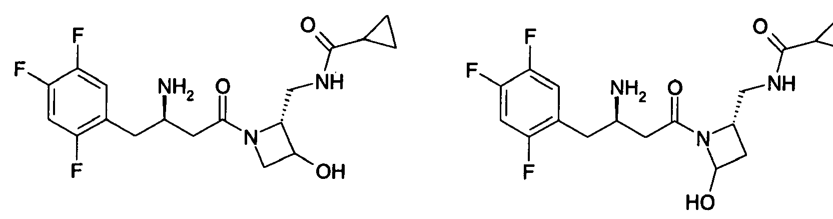

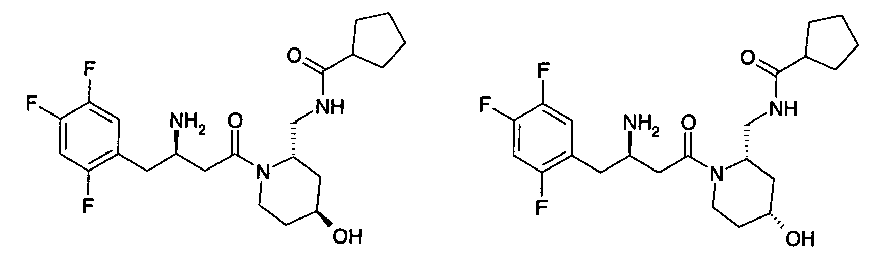

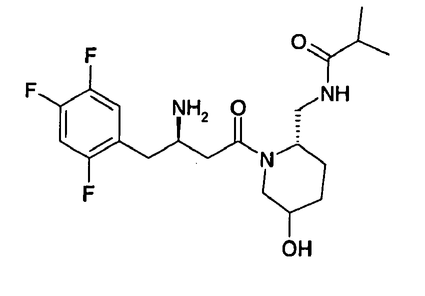

The invention further relates to a compound of formula (II) 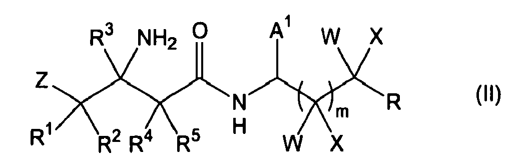 or a pharmaceutically acceptable salt thereof, wherein
Z is selected from the group consisting of
phenyl;
naphthyl;
C₃₋₇ cycloalkyl;
heterocycle; and
heterobicycle;
   wherein Z is optionally substituted with one, or independently from each other, more of
   halogen;
   CN;
   OH;
   =O, where the ring is at least partially saturated;
   C₁₋₆ alkyl, optionally substituted with one or more F; and
   O-C₁₋₆ alkyl, optionally substituted with one or more F;
R¹, R², R⁴ and R⁵ are independently from each other selected from the group consisting of
H;
F;
OH;
C₁₋₆ alkyl, optionally substituted with one or more F; and
O-C₁₋₆ alkyl, optionally substituted with one or more F;
and/or R¹ and R² optionally form together C₃₋₇ cycloalkyl, which is optionally substituted with one or more F;
and/or R² and R³ optionally form together C₃₋₇ cycloalkyl, which is optionally substituted with one or more F;
and/or R³ and R⁴ optionally form together C₃₋₇ cycloalkyl, which is optionally substituted with one or more F;
and/or R⁴ and R⁵ optionally form together C₃₋₇ cycloalkyl, which is optionally substituted with one or more F;
R³ is H or C₁₋₆ alkyl;
R is
COOH or NR'R" and
   wherein R' and R" are independently from each other selected from
   H; and
   C₁₋₄ alkyl, optionally substituted with one or more F;
or R' and R" may form together a C₃₋₇ cycloalkyl, which is optionally substituted with one or more F;
X are independently from each other selected from
H;
F;
CH₃;
OCH₃;
OH;
W are independently from each other H or F;
m is 0,1 or 2;
A¹ is -C(R⁶R⁷)-Y-T;
R⁶ and R⁷ are independently from each other selected from the group consisting of H;
F; and
C₁₋₆ alkyl, optionally substituted with one or more F;
or R⁶ and R⁷ optionally form together C₃₋₇ cycloalkyl, which is optionally substituted with one or more F;
Y is selected from the group consisting of
-O-;
-C₁₋₆ alkyl-O-;
-N(R⁸)-;
-C₁₋₆ alkyl-N(R⁸)-;
-S-;
-C₁₋₆ alkyl-S-;
-S(O)-;
-C₁₋₆ alkyl-S(O)-;
-S(O)₂-; and
-C₁₋₆ alkyl-S(O)₂-;
   wherein each C₁₋₆ alkyl is optionally substituted with one or more F;
R⁸ and T are independently from each other T¹-T² or T²;
T¹ is selected from the group consisting of
-C₁₋₆ alkyl-;
-C₁₋₆ alkyl-O-;
-C₁₋₆ alkyl-N(R⁹)-;
-C(O)-;
-C(O)-C₁₋₆ alkyl-;
-C(O)-C₁₋₆ alkyl-O-;
-C(O)-C₁₋₆ alkyl-N(R⁹)-;
-C(O)O-;
-C(O)O-C₁₋₆ alkyl-;
-C(O)O-C₁₋₆ alkyl-O-;
-C(O)O-C₁₋₆ alkyl-N(R⁹)-;
-C(O)N(R⁹)-;
-C(O)N(R⁹)-C₁₋₆ alkyl-;
-C(O)N(R⁹)-C₁₋₆ alkyl-O-;
-C(O)N(R⁹)-C₁₋₆ alkyl-N(R¹⁰)-;
-S(O)₂-;
-S(O)₂-C₁₋₆ alkyl-;
-S(O)₂-C₁₋₆ alkyl-O-; and
-S(O)₂-C₁₋₆ alkyl-N(R⁹)-;
   wherein each C₁₋₆ alkyl is optionally substituted with one or more F;
R⁹ and R¹⁰ are independently from each other H or C₁₋₆ alkyl, optionally substituted with one or more F;
T² is selected from the group consisting of
H;
CF₃;
phenyl;
naphthyl;
   wherein phenyl and naphthyl are optionally substituted with one, or independently from each other, more of
   halogen;
   CN;
   R¹¹;
   COOH;
   OH;
   C(O)NH₂;
   S(O)₂NH₂;
   COOT³;
   OT³;
   C(O)NHT³;
   S(O)₂NHT³; or
   T³;
C₃₋₇ cycloalkyl;
heterocycle; and
heterobicycle;
   wherein C₃₋₇ cycloalkyl, heterocycle and heterobicycle are optionally substituted with one, or independently from each other, more of
   halogen;
   CN;
   R¹²;
   OH;
   =O, where the ring is at least partially saturated;
   NH₂;
   COOH;
   C(O)NH₂;
   S(O)₂NH₂;
   COOT³;
   OT³;
   C(O)NHT³;
   S(O)₂NHT³;
   NHT³; or
   T³;
whereby when R⁸ is T¹-T² and represents -C₁₋₆ alkyl and T is T¹-T² and represents - C₁₋₆ alkyl then R⁹ and T may form together a 3 to 7 membered cyclic group containing one nitrogen atom;
R¹¹ is selected from the group consisting of
C₁₋₆ alkyl;
O-C₁₋₆ alkyl;
COO-C₁₋₆ alkyl;
OC(O)-C₁₋₆ alkyl;
C(O)N(R¹⁴)-C₁₋₆ alkyl;
S(O)₂N(R¹⁶)-C₁₋₆ alkyl;
S(O)-C₁₋₆ alkyl;
S(O)₂-C₁₋₆ alkyl; and
N(R¹⁷)S(O)-C₁₋₆ alkyl;
   wherein each C₁₋₆ alkyl is optionally substituted with one, or independently from each other, more of F, COOR¹⁸, C(O)N(R¹⁹R²⁰), S(O)₂N(R²¹R²²), OR²³, N(R²⁴R²⁵), T³, O-T³ or N(R²⁶)-T³;
R¹² is selected from the group consisting of
C₁₋₆ alkyl;
O-C₁₋₆ alkyl;
N(R¹³)-C₁₋₆ alkyl;
COO-C₁₋₆ alkyl;
OC(O)-C₁₋₆ alkyl;
C(O)N(R¹⁴)-C₁₋₆ alkyl;
N(R¹⁵)-C(O)-C₁₋₆ alkyl;
S(O)₂N(R¹⁶)-C₁₋₆ alkyl;
S(O)-C₁₋₆ alkyl;
S(O)₂-C₁₋₆ alkyl; and
-N(R¹⁷)S(O)₂-C₁₋₆ alkyl;
   wherein each C₁₋₆ alkyl is optionally substituted with one, or independently from each other, more of F, COOR¹⁸, C(O)N(R¹⁹R²⁰), S(O)₂N(R²¹R²²), OR²³, N(R²⁴R²⁵), T³, O-T³ or N(R²⁶)-T³;
R¹³, R¹⁴, R¹⁵, R¹⁶, R¹⁷, R¹⁸, R¹⁹, R²⁰, R²¹, R²², R²³, R²⁴, R²⁵ and R²⁶ are independently from each other H or C₁₋₆ alkyl;
T³ is selected from the group consisting of
phenyl;
naphthyl;
   wherein phenyl and naphthyl are optionally substituted with one, or independently from each other, more of
   halogen;
   CN;
   COOH;
   OH;
   C(O)NH₂;
   S(O)₂NH₂;
   C₁₋₆ alkyl;
   O-C₁₋₆ alkyl;
   COO-C₁₋₆ alkyl;
   OC(O)-C₁₋₆ alkyl;
   C(O)N(R²⁷)-C₁₋₆ alkyl;
   S(O)₂N(R²⁸)-C₁₋₆ alkyl;
   S(O)₂-C₁₋₆ alkyl; or
   N(R²⁹)S(O)₂-C₁₋₆ alkyl;
heterocycle;
heterobicycle; and
C₃₋₇ cycloalkyl;
   wherein C₃₋₇ cycloalkyl, heterocycle and heterobicycle are optionally substituted with one, or independently from each other, more of
   halogen;
   CN;
   OH;
   =O, where the ring is at least partially saturated;
   NH₂
   COOH;
   C(O)NH₂;
   S(O)₂NH₂;
   C₁₋₆ alkyl;
   O-C₁₋₆ alkyl;
   N(R³⁰)-C₁₋₆ alkyl;
   COO-C₁₋₆ alkyl;
   OC(O)-C₁₋₆ alkyl;
   C(O)N(R³¹)-C₁₋₆ alkyl;
   N(R³²)-C(O)-C₁₋₆ alkyl;
   S(O)₂N(R³³)-C₁₋₆ alkyl;
   S(O)₂-C₁₋₆ alkyl; or
   -N(R³⁴)S(O)₂-C₁₋₆ alkyl;
   and
R²⁷, R²⁸, R²⁹, R³⁰, R³¹, R³², R³³ and R³⁴ are independently from each other H or C₁₋₆ alkyl.

A preferred stereochemistry of compounds according to the present invention is shown in formula (IIa)

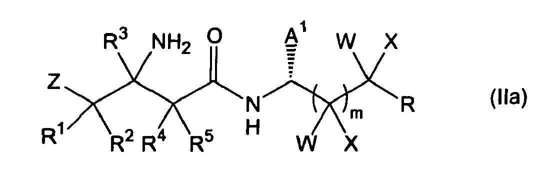

Preferred compounds of formula (II) or (IIa) are those compounds in which one or more of the residues contained therein have the meanings given below. It is understood, that the claimed compounds cover any compound obtained by combining any of the definitions disclosed within this description for the various substituents. With respect to all preferred compounds of the formulas (II) or (IIa) the present invention also includes all tautomeric and stereoisomeric forms and mixtures thereof in all ratios, and their pharmaceutically acceptable salts.

In preferred embodiments of the present invention, the substituents R¹ - R⁵, Z, X, m and A¹ of the formula (II) or (IIa) independently from each other have the following meaning. Hence, one or more of the substituents R¹ - R⁵, Z, X, m and A¹ can have the preferred or more preferred meanings given below.

Z is preferably is phenyl or heterocycle and Z is optionally substituted independently from each other with 1, 2 or 3, more preferably up to 2 or 3, of Cl, F, CN, CH₃ or OCH₃. In one embodiment Z is substituted with up to 3 F.

It is preferred that R¹, R², R⁴ and R⁵ are independently from each other selected from the group consisting of H, F, OH, CH₃, and OCH₃.

R³ is preferably H.

X is preferably H or F.

W is preferably H. In another embodiment, W and X both represent F. In a preferred embodiment, W and X both represent F and are both attached to the same carbon atom.

Preferably, m is 1.

Y is preferably -O-, -N(R⁸)- or -S(O)₂-, more preferably -O- or -N(R⁸)-. Most preferably, Y is -N(R⁸)-.

Preferably, R⁸ is selected from the group consisting of H, CH₃, COOH, COOCH₃, C(O)NH₂, C(O)N(CH₃)₂, and S(O)₂CH₃, more preferably H and CH₃, most preferably H.

It is preferred that T is T¹-T² or T², wherein T¹ is selected from the group consisting of
-CH₂-;
-C(O)-;
-C(O)-CH₂-;
-C(O)O-;
-C(O)O-CH₂-;
-C(O)NH-;
-C(O)NH-CH₂-;
-S(O)₂-; and
-S(O)₂-CH₂-.
More preferred is T¹ selected from the group consisting of -C(O)-, -CH₂-, -S(O)₂- and -C(O)NH-.

It is preferred that T is T¹-T². In this case, T¹-T² is preferably a group as defined below.

In one embodiment, T¹-T² is preferably CH₂-phenyl, whereby phenyl may be substituted with 1 to 3, preferably 1 or 2, substituents selected from halogen, CN, O-C₁₋₄ alkyl, C₁₋₄ alkyl or S(O)₂CH₃, preferably F, Cl, O-Me, Me or S(O)₂CH₃.
In one embodiment, T¹-T² is preferably CH₂-C₃₋₇ cycloalkyl, more preferably cyclopropyl or cyclobutyl, more preferably cyclopropyl, whereby cycloalkyl may be substituted with 1 or 2, preferably 1, substituents selected from halogen, CN, OH, NH₂, COOH, C(O)NH₂ or S(O)₂NH₂, more preferably COOH or C(O)NH₂.
In one embodiment, T¹-T² is preferably C₁₋₄ alkyl, preferably methyl, ethyl or propyl, most preferably methyl.
In one embodiment, T¹-T² is preferably C(O)-phenyl, whereby phenyl may be substituted with 1 to 3, preferably 1 or 2, substituents selected from halogen, CN, O-C₁₋₄alkyl, C₁₋₄alkyl or S(O)₂CH₃, preferably F, Cl, O-Me, Me or S(O)₂CH₃.
In one embodiment, T¹-T² is preferably C(O)-C₃₋₇ cycloalkyl, more preferably cyclopropyl or cyclobutyl, more preferably cyclopropyl, whereby cycloalkyl may be substituted with 1 to 3, preferably 1 or 2, substituents selected from halogen, CN, O-C₁₋₄ alkyl, C₁₋₄ alkyl, whereby alkyl may be further substituted with 1 to 3 F; more preferably cycloalkyl may be substituted with one C₁₋₄ alkyl substituted with 1 to 3 F.
In one embodiment, T¹-T² is preferably C(O)-heterocycle, whereby heterocycle may be substituted with 1-3, preferably 1 or 2, substituents selected from halogen, CN, O-C₁₋₄ alkyl, C₁₋₄ alkyl or S(O)₂CH₃; preferably, the heterocycle is aromatic, more preferably containing 1 or 2 heteroatoms selected from N and O, most preferably N.
In one embodiment, T¹-T² is preferably S(O)₂-phenyl, whereby phenyl may be substituted with 1-3, preferably 1 or 2, substituents selected from halogen, CN, O-C₁₋₄ alkyl, C₁₋₄ alkyl or S(O)₂CH₃, preferably F, Cl, O-Me, Me or S(O)₂CH₃.
In one embodiment, T¹-T² is preferably S(O)₂-C₃₋₇ cycloalkyl, more preferably cyclopropyl or cyclobutyl, more preferably cyclopropyl, whereby cycloalkyl may be substituted with 1-3, preferably 1 or 2, substituents selected from halogen, CN, O-C₁₋₄ alkyl, C₁₋₄ alkyl, whereby alkyl may be further substituted with 1 to 3 F; more preferably cycloalkyl may be substituted with one C₁₋₄ alkyl substituted with 1 to 3 F.
In one embodiment, T¹-T² is preferably S(O)₂-C₁₋₄ alkyl, preferably S(O)₂CH₃.
In one embodiment, T¹-T² is preferably C(O)-NH-phenyl, whereby phenyl may be substituted with 1-3, preferably 1 or 2, substituents selected from halogen, CN, O-C₁₋₄ alkyl, C₁₄ alkyl or S(O)₂CH₃.

When T is T², is preferably a group as defined below.

In one embodiment, T² is preferably H.
In one embodiment, T² is preferably phenyl, whereby phenyl may be substituted with 1-3, preferably 1 or 2, substituents selected from halogen, CN, O-C₁₋₄ alkyl, C₁₋₄ alkyl or S(O)₂CH₃, preferably F, Cl, O-Me, Me or S(O)₂CH₃,
In one embodiment, T² is preferably heterocycle, whereby heterocycle may be substituted with 1-3, preferably 1 or 2, substituents selected from halogen, CN, phenyl, heterocycle, O-C₁₋₄ alkyl, C₁₋₄ alkyl or S(O)₂CH₃; preferably, the heterocycle is aromatic, more preferably containing 1, 2 or 3 heteroatoms selected from N and O, most preferably N. When the heterocycle is substituted with phenyl or heterocycle, the heterocycle is preferably aromatic, more preferably containing 1, 2 or 3 heteroatoms selected from N and O, most preferably N, and the phenyl or heterocycle may be further substituted by 1 or 2 F or S(O)₂CH₃.

In one embodiment, T² is preferably CF₃.

T² is preferably phenyl or heterocycle.

Preferably, A¹ is -CH₂-N(R⁸)-T, wherein R⁸ is H, S(O)₂CH₃ or S(O)₂-C₃₋₇ cycloalkyl, most preferably H.

In other embodiments, A¹ is -CH₂-O-T.

In the case that Y contains the group R⁸, the following is preferred in embodiments:
When R⁸ is T¹-T² and represents -C₁₋₆ alkyl and T is T¹-T² and represents -C₁₋₆ alkyl then R⁸ and T may form together a 3 to 7 membered cyclic group containing one nitrogen atom, preferably a 5 or 6 membered cyclic group.

Preferred compounds of formula (II) or (IIa) are shown below.

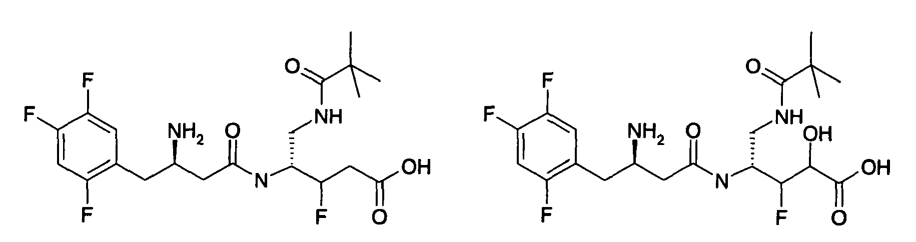

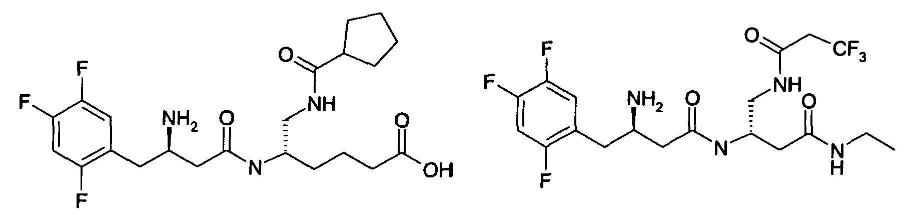

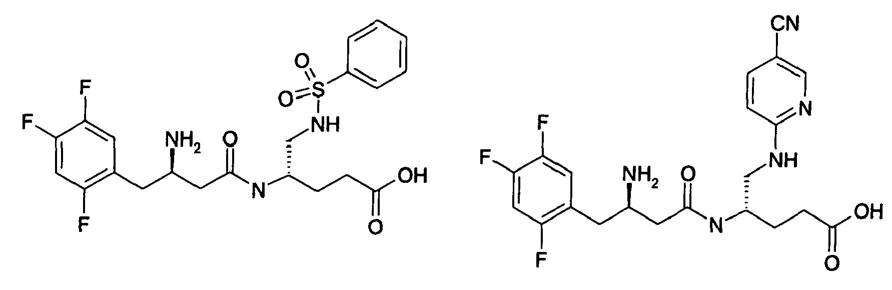

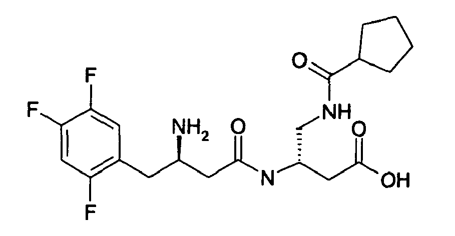

Furthermore, the present invention provides prodrug compounds of the compounds of the invention as described above.

"Prodrug compound" means a derivative that is converted into a compound according to the present invention by a reaction with an enzyme, gastric acid or the like under a physiological condition in the living body, e.g. by oxidation, reduction, hydrolysis or the like, each of which is carried out enzymatically. Examples of the prodrug are compounds, wherein the amino group in a compound of the present invention is acylated, alkylated or phosphorylated to form, e.g., eicosanoylamino, alanylamino, pivaloyloxymethylamino or wherein the hydroxyl group is acylated, alkylated, phosphorylated or converted into the borate, e.g. acetyloxy, palmitoyloxy, pivaloyloxy, succinyloxy, fumaryloxy, alanyloxy or wherein the carboxyl group is esterified or amidated. These compounds can be produced from compounds of the present invention according to well-known methods.

Metabolites of compounds of formula (I), (Ia), (II) and (IIa) are also within the scope of the present invention.

Where tautomerism, like e.g. keto-enol tautomerism, of compounds of general formula (I), (Ia), (II) or (IIa) or their prodrugs may occur, the individual forms, like e.g. the keto and enol form, and together as mixtures in any ratio are also within the scope of the invention. Same applies for stereoisomers, like e.g. enantiomers, cis/trans isomers, conformers and the like.
If desired, isomers can be separated by methods well known in the art, e.g. by liquid chromatography. Same applies for enantiomers by using e.g. chiral stationary phases. Additionally, enantiomers may be isolated by converting them into diastereomers, i.e. coupling with an enantiomerically pure auxiliary compound, subsequent separation of the resulting diastereomers and cleavage of the auxiliary residue. Alternatively, any enantiomer of a compound of formula (I), (Ia), (II) or (IIa) may be obtained from stereoselective synthesis using optically pure starting materials.

In case the compounds according to formula (I), (Ia), (II) or (IIa) contain one or more acidic or basic groups, the invention also comprises their corresponding pharmaceutically or toxicologically acceptable salts, in particular their pharmaceutically utilizable salts. Thus, the compounds of the formula (I), (Ia), (II) or (IIa) which contain acidic groups can be present on these groups and can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Compounds of the formula (I), (la), (II) or (IIa) which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the formula (I), (la), (II) or (IIa) simultaneously contain acidic and basic groups in the molecule, the invention also includes, in addition to the salt forms mentioned, inner salts or betaines (zwitterions). The respective salts according to the formula (I), (la), (II) or (IIa) can be obtained by customary methods which are known to the person skilled in the art like, for example by contacting these with an organic or inorganic acid or base in a solvent or dispersant, or by anion exchange or cation exchange with other salts. The present invention also includes all salts of the compounds of the formula (I), (la), (II) or (IIa) which, owing to low physiological compatibility, are not directly suitable for use in pharmaceuticals but which can be used, for example, as intermediates for chemical reactions or for the preparation of pharmaceutically acceptable salts.

Within the meaning of the present invention the terms are used as follows:
"C₁₋₆ Alkyl" means a straight-chain or branched carbon chain having 1 - 6 carbon atoms, e.g. methyl, ethyl, n-propyl, isopropyl, n-butyl, isobutyl, sec-butyl, tert-butyl, n-pentane, or n-hexane. If the alkyl group is used as a bridging moiety between two substituents, the term "alkyl" comprises e.g. -CH₂-, -CH₂-CH₂-, -CH(CH₃)-, -C(CH₂)-, -CH₂-CH₂-CH₂-, -CH(C₂H₅)-, or -CH(CH₃)₂-. Each hydrogen of a C₁₋₆ alkyl carbon may be replaced by a substituent.

"C₃₋₇ Cycloalkyl" means a cyclic alkyl chain having 3-7 carbon atoms, e.g. cyclopropyl, cyclobutyl, cyclopentyl, cyclohexyl, cycloheptyl. Each hydrogen of a cycloalkyl carbon may be replaced by a substituent.

"Halogen" means fluoro, chloro, bromo or iodo. It is generally preferred that halogen is fluoro or chloro.

"Heterocycle" means a cyclopentane, cyclohexane or cycloheptane ring that may contain up to the maximum number of double bonds (aromatic or non-aromatic ring which is fully, partially or un-saturated) wherein at least one carbon atom up to 4 carbon atoms are replaced by a heteroatom selected from the group consisting of sulfur (including -S(O)-, -S(O)₂-), oxygen and nitrogen (including =N(O)- and wherein the ring is linked to the rest of the molecule via a carbon or nitrogen atom. Examples for a heterocycle are furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, azepine or homopiperazine.

"Heterobicycle" means a heterocycle which is condensed with phenyl or an additional heterocycle to form a bicyclic ring system. "Condensed" to form a bicyclic ring means that two rings are attached to each other by sharing two ring atoms. Examples for a heterobicycle are indole, indoline, benzofuran, benzothiophene, benzoxazole, benzisoxazole, benzothiazole, benzisothiazole, benzimidazole, benzimidazoline, quinoline, quinazoline, dihydroquinazoline, dihydroquinoline, isoquinoline, tetrahydroisoquinoline, dihydroisoquinoline, benzazepine, purine or pteridine.

The present invention provides compounds of general formula (I), (la), (II) or (11a) or their prodrugs as DPP-IV inhibitors. DPP-IV is a cell surface protein that has been implicated in a wide range of biological functions. It has a broad tissue distribution (intestine, kidney, liver, pancreas, placenta, thymus, spleen, epithelial cells, vascular endothelium, lymphoid and myeloid cells, serum), and distinct tissue and cell-type expression levels. DPP-IV is identical to the T cell activation marker CD26, and it can cleave a number of immunoregulatory, endocrine, and neurological peptides *in vitro.* This has suggested a potential role for this peptidase in a variety of disease processes.

DPP-IV related diseases are described in more detail in WO-A-03/000181 under the paragraph "Utilities" which is herewith incorporated by reference.

Accordingly, the present invention provides compounds of formula (I), (la), (II) or (IIa) or their prodrugs or pharmaceutically acceptable salt thereof for use as a medicament.

Furthermore, the present invention provides the use of compounds of formula (I), (Ia), (II) or (IIa) or their prodrugs or a pharmaceutically acceptable salt thereof for the manufacture of a medicament for the treatment or prophylaxis of non-insulin dependent (Type II) diabetes mellitus; hyperglycemia; obesity; insulin resistance; lipid disorders; dyslipidemia; hyperlipidemia; hypertriglyceridemia; hypercholestrerolemia; low HDL; high LDL; atherosclerosis; growth hormone deficiency; diseases related to the immune response; HIV infection; neutropenia; neuronal disorders; anxiety; depression; tumor metastasis; benign prostatic hypertrophy; gingivitis; hypertension; osteoporosis; diseases related to sperm motility; low glucose tolerance; insulin resistance; ist sequelae; vascular restenosis; irritable bowel syndrome; inflammatory bowel disease; including Crohn's disease and ulcerative colitis; other inflammatory conditions; pancreatitis; abdominal obesity; neurodegenerative disease; retinopathy; nephropathy; neuropathy; Syndrome X; ovarian hyperandrogenism (polycystic ovarian syndrome; Type n diabetes; or growth hormone deficiency. Preferred is non-insulin dependent (Type II) diabetes mellitus and obesity.

The present invention provides pharmaceutical compositions comprising at least one compound of formula (I), (Ia), (II) or (IIa), a prodrug compound thereof, or a pharmaceutically acceptable salt thereof as active ingredient together with a pharmaceutically acceptable carrier.

"Pharmaceutical composition" means one or more active ingredients, and one or more inert ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of the present invention and a pharmaceutically acceptable carrier.

A pharmaceutical composition of the present invention may additionally comprise one or more other compounds as active ingredients like other DPP-IV inhibitors.
Other active ingredients are disclosed in WO-A-03/000181 under the paragraph "Combination Therapy" which is herewith incorporated by reference.
Accordingly, other active ingredients may be insulin sensitizers; PPAR agonists; biguanides; protein tyrosinephosphatase-IB (PTP-1 B) inhibitors; insulin and insulin mimetics; sulfonylureas and other insulin secretagogues; a-glucosidase inhibitors; glucagon receptor antagonists; GLP-1, GLP-1 mimetics, and GLP-1 receptor agonists; GIP, GIP mimetics, and GIP receptor agonists; PACAP, PACAP mimetics, and PACAP receptor 3 agonists; cholesterol lowering agents; HMG-CoA reductase inhibitors; sequestrants; nicotinyl alcohol; nicotinic acid or a salt thereof; PPARa agonists; PPARoly dual agonists; inhibitors of cholesterol absorption; acyl CoA : cholesterol acyltransferase inhibitors; anti-oxidants; PPARo agonists; antiobesity compounds; an ileal bile acid transporter inhibitor; or anti-inflammatory agents or pharmaceutically acceptable salts of these active compounds.

The term "pharmaceutically acceptable salts" refers to salts prepared from pharmaceutically acceptable non-toxic bases or acids, including inorganic bases or acids and organic bases or acids.

The compositions include compositions suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

In practical use, the compounds of formula (I), (Ia), (II) or (IIa) can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, hard and soft capsules and tablets, with the solid oral preparations being preferred over the liquid preparations.

Because of their ease of administration, tablets and capsules represent the most advantageous oral dosage unit form in which case solid pharmaceutical carriers are obviously employed. If desired, tablets may be coated by standard aqueous or nonaqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally as, for example, liquid drops or spray.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin.

When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

Compounds of formula (I), (Ia), (II) or (IIa) may also be administered parenterally. Solutions or suspensions of these active compounds can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (*e.g*., glycerol, propylene glycol and liquid polyethylene glycol), suitable mixtures thereof, and vegetable oils.
Any suitable route of administration may be employed for providing a mammal, especially a human, with an effective dose of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably compounds of formula (I), (Ia), (II) or (IIa) are administered orally.

The effective dosage of active ingredient employed may vary depending on the particular compound employed, the mode of administration, the condition being treated and the severity of the condition being treated. Such dosage may be ascertained readily by a person skilled in the art.

When treating or preventing diabetes mellitus and/or hyperglycemia or hypertriglyceridemia or other diseases for which compounds of Formula I are indicated, generally satisfactory results are obtained when the compounds of the present invention are administered at a daily dosage of from about 0.1 milligram to about 100 milligram per kilogram of animal body weight, preferably given as a single daily dose or in divided doses two to six times a day, or in sustained release form. For most large mammals, the total daily dosage is from about 1.0 milligrams to about 1000 milligrams, preferably from about 1 milligrams to about 50 milligrams. In the case of a 70 kg adult human, the total daily dose will generally be from about 7 milligrams to about 350 milligrams. This dosage regimen may be adjusted to provide the optimal therapeutic response.

The compounds of formula (I) or (II) of the present invention can be prepared from beta amino acid intermediates such as those of formula (XVIII) and substituted amine intermediates such as those of formula (IX) and (XVII), using standard peptide coupling conditions followed by deprotection reactions. The preparation of these intermediates is described in the following schemes.

Some abbreviations that may appear in this application are as follows.

### ABBREVIATIONS

### Designation

- br: Broad signal
- Boc (or BOC): *tert*-Butoxycarbonyl
- CDI: *N*,*N*-Carbonyldiimidazole
- DCE: 1,2-Dichloroethane
- DCM: Dichloromethane
- DIEA: Diisopropylethylamine
- DMF: *N,N*-Dimethylformamide
- EDC: 1-Ethyl-3-(3-dimethylaminopropyl)carbodiimide hydrochloride
- NEt₃: Triethylamine
- Fmoc: 9-Fluorenylmethoxycarbonyl
- HATU: *O*-(7-Azabenzotriazo)-1-yl)-*N,N,N',N'*-tetramethyluronium hexafluorophosphate
- HCl: Hydrogen chloride
- HOBt: 1-Hydroxybenzotriazole
- HPLC: High pressure liquid chromatography
- M.P.: Melting point
- NMR: Nuclear Magnetic Resonance
- PG: Protecting group
- rt: Retention time
- ^{t}BuOH: *tert*-Butanol
- TBAF: Tetra-n-butylammonium fluoride
- TFA: Trifluoroacetic acid
- TLC: Thin Layer Chromatography

In the following schemes A to H as well as in compounds (III) to (VI) the variants W and X have been omitted for clarity reasons.

Available starting materials may be suitably *N*-protected amino acids (III-VI, R = H; PG¹ = protecting group), or amino esters (III-VI, R = alkyl; PG¹ = protecting group).

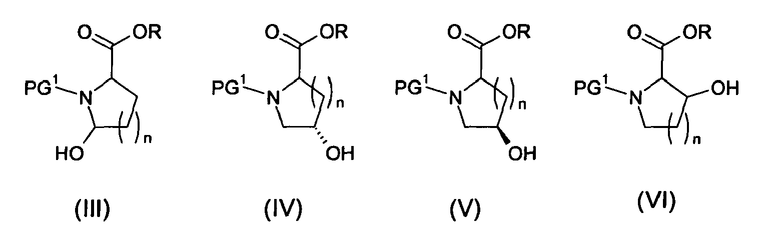

They may be purchased from commercially available sources such as ABCR, Array, Astatech, Sigma-Aldrich, Fluka or be synthesised by:
*(a) Amide reduction.* According to Scheme A compounds of the type (III) may be prepared for example from commercially available 5-oxo-proline (n = 1). First the acid is transformed into a suitable ester (R = alkyl), and the amide is protected with an carbamate group (e.g. Boc). The resulting compound can be reduced to the N-carbamate half aminal by reduction with diisobutylaluminum hydride in the presence of sodium, potassium tartrate in aqueous THF (see Corey et al, J. Org. Chem. 1990, 784-786.) 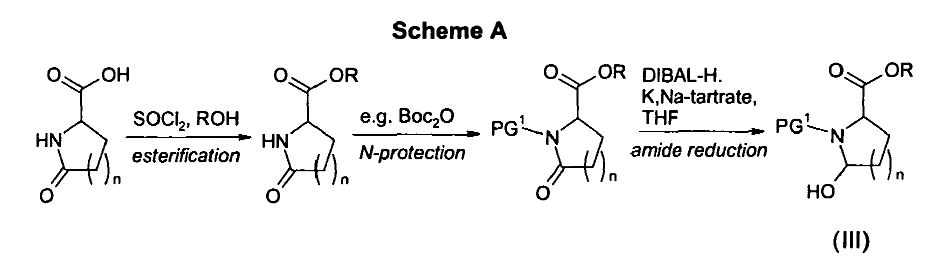
*(b) Ketone reduction.* Compounds of the type (IV-VI) may be produced by reduction of the corresponding ketone with hydride reagents, as shown in Scheme B and C. 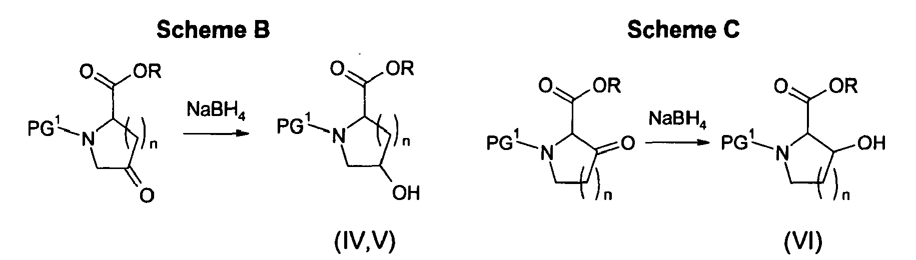 Suitable starting materials for keto compounds for Scheme C are mostly commercial or may be prepared by either a Dieckmann Cyclization, as described in Pellegrini, et al Tetrahedron Lett. 2002, 3243-3246 or by a rhodium mediated ring closing reaction, as reported by Moyer, et al in J. Org. Chem. 1985, 5223-5230 as shown in Scheme D.

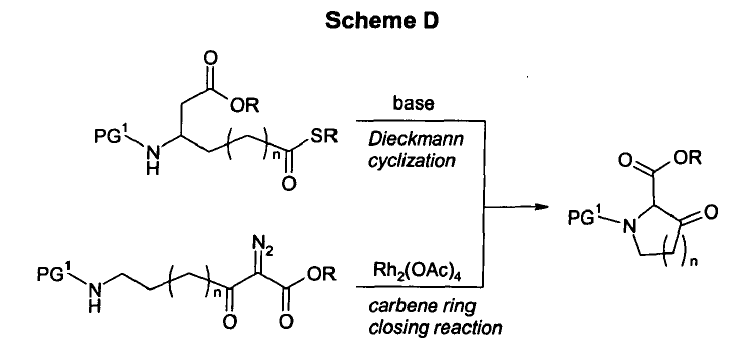

Commencing from alcohols (III-VI), diamines (VII) may be prepared by the reaction sequence shown in Scheme E. In the first step the alcohol is protected with a suitable protecting group (PG²), preferably a silyl group. For a comprehensive list of alcohol protecting groups see: Greene, Wuts, Protective Groups in Organic Synthesis, 3rd ed., 1999, Wiley & Sons, New York.

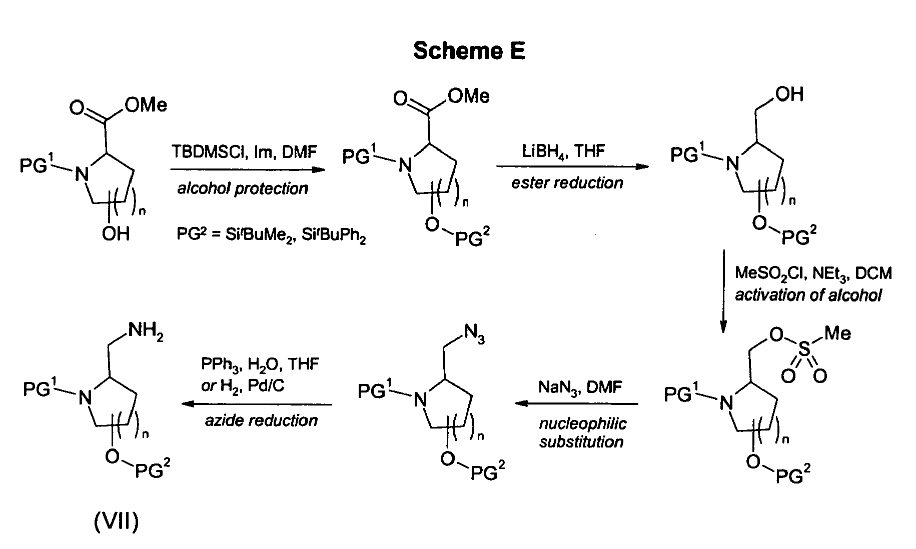

The reaction sequence further comprises the reduction of the ester, followed by transformation of the resulting alcohol into a good leaving group and subsequent nucleophilic displacement with sodium azide. Alternatively, a *Gabriel synthesis* may be performed to synthesize compounds of the type (VII), which uses potassium phthalimide as nucleophile and hydrazine to liberate the primary amine. The azide may be reduced to the primary amine (VII) by either a *Staudinger reaction* with triphenylphosphane (see: Tian, Wang, J. Org. Chem. 2004, 69, 4299. or Lin, et al J. Am. Chem. Soc, 2005, 127, 2686.) or hydrogen reduction with palladium on charcoal as catalyst.

In the following, primary amines of the type (VII) may be reacted with a carboxylic acid under amide coupling conditions with e.g. EDC, HOBt and a base (e.g. triethylamine) to form amides of the type (VIII). Sulfonamides of the type (IX) can result from the reaction of diamines (VII) with sulfonylchlorides and amines of the type (X) can be the product of a nucleophilic substitution reaction of diamine (VII) with activated aryl halogenides or alkyl halogenides as shown in Scheme F or other suitable methods.

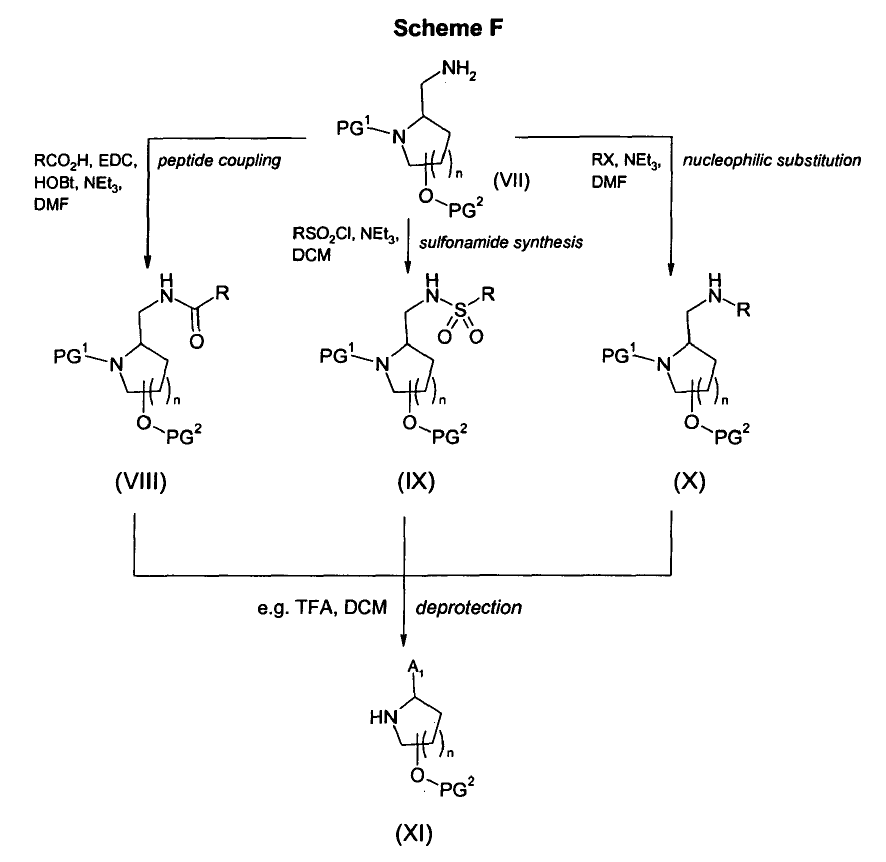

Final deprotection of (VIII-X) with e.g. trifluoroacetic acid in the case of PG¹ = Boc yields amine building blocks (XI) which are used to prepare title compounds (I) of the present invention.

An example for the first steps of the synthesis of amine building blocks (XVII) are shown in Scheme G. Commencing from suitably protected amino acids (aspartic acid derivative, n = 1; glutamic acid derivative, n = 2), the acid is reduced to the alcohol in the first step by reaction with carbonyl diimidazole followed by sodium borohydride reduction.

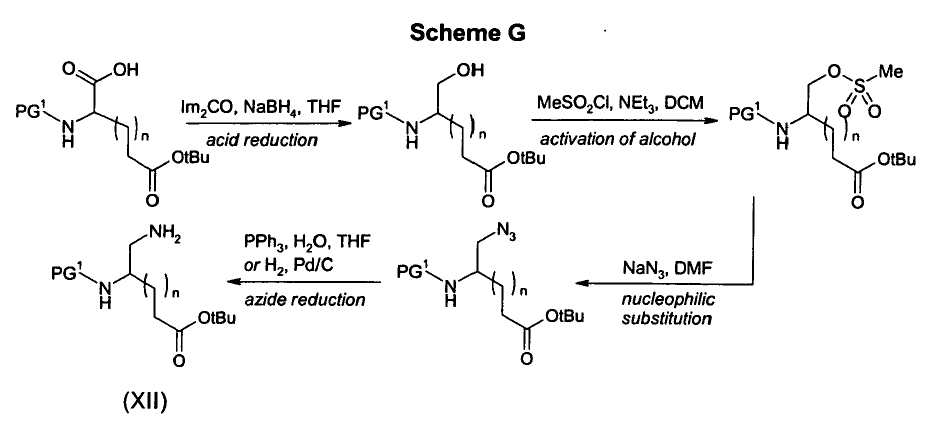

Subsequently the alcohol may be converted into the azide after activation with, e.g. methane sulfonylchloride, followed by a nucleophilic substitution with sodium azide. The azide can be transformed into the primary amine (XII) by either the *Staudinger reaction* (triphenylphosphine, water) or catalytic hydrogenation. Alternatively, the Gabriel sequence may be employed to yield diamines of the type (XII), as described earlier.

In the following, primary amines of the type (XII) may be reacted with a carboxylic acid under amide coupling conditions with e.g. EDC, HOBt and a base (e.g. triethylamine) to form amides of the type (XIII). Sulfonamides of the type (XIV) can result from the reaction of diamines (XII) with sulfonylchlorides and amines of the type (XV) can be the product of a nucleophilic substitution reaction of diamine (XII) with activated aryl halogenides or alkyl halogenides as shown in Scheme H.

Final deprotection of (XIII-XV) with e.g. trifluoroacetic acid in the case of PG¹ = Boc yields amino acids (XVI) which can be transformed into the corresponding esters by reaction with thionyl chloride and an alcohol, preferably methanol or ethanol. Amino building blocks (XVII) which are synthesized by this sequence may be used to prepare title compounds (II) of the present invention.

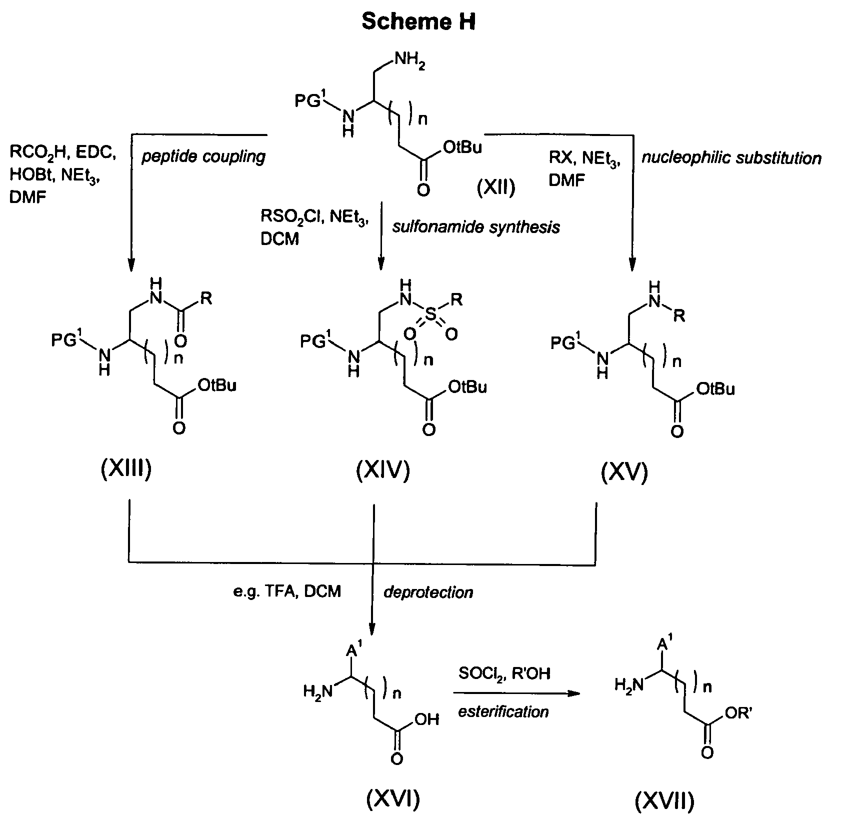

Enantiomerically pure beta amino acids having the formula (XVIII) as used in Schemes J and K 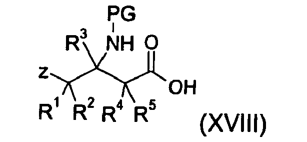 may be commercially available, are known in the literature or may be conveniently synthesized using one of the methods already published and reviewed in e.g., Cole, Tetrahedron 1994, 32, 9517, Juaristi et al., Aldrichimica Acta 1994, 27, 3, or Juaristi, Enantioselective Synthesis of β-Amino Acids, Ed. Wiley-VCH, New York, 1997. In particular, 3-amino-4-(2,4,5-trifluoro-phenyl)-butyric acid may be synthesized by a variety of methods as reported in the patent applications WO 2004069162, WO 2004064778, WO 2004037169, WO 2004032836 and in the articles J. Am. Chem. Soc. 2004, 126, 3048 and J. Am. Chem. Soc. 2004, 126, 9918.

Unless otherwise noted, all non-aqueous reactions were carried out under argon atmosphere with commercial dry solvents. Compounds were purified using flash column chromatography using Merck silica gel 60 (230-400 mesh) or reverse phase preparative HPLC using a Reprosil-Pur ODS3, 5 µm, 20 x 125 mm column with Shimadzu LCBA-Pump and SPD-10Avp UV/Vis diode array detector. The ¹H-NMR spectra were recorded on a Varian VXR-S (300 MHz for ¹H-NMR) using d₆-dimethylsulfoxide as solvent; chemical shifts are reported in ppm relative to tetramethylsilane. Analytical LC/MS was performed using Reprosil-Pur ODS3, 5 µM, 1 x 60 mm columns with a linear gradient from 5% to 95% acetonitrile in water (0.1% formic acid) at a flow rate of 250 µl/min; retention times are given in minutes. Methods are run on:
(I) LC10Advp-Pump (Shimadzu) with SPD-M10Avp UV/Vis diode array detector and QP2010 MS-detector in ESI+ modus with UV-detection at 214, 254 and 275 nm, with a linear gradient of acetonitrile in water (0.1 % formic acid). The data will be reported as follows: LC/MS (I) (5-95%, 5 min): rt 1.60, m/z 171 [M+H].
(II) LC10Advp-Pump (Shimadzu) with SPD-10Avp dual wavelength UV-detector and QP2010 MS-detector in ESI+ modus with UV-detection at 214 and 254 nm, with a 10 min. linear gradient of acetonitrile in water (0.1% formic acid) followed by washing the column (99% acetonitrile) and data-aquisition until 15 min. The data will be reported as follows: LC/MS (II) (1-30%, 10 min): rt 1.60, m/z 171 [M+H].

### General procedure for making compounds of the invention

In general, compounds having the formula (I) 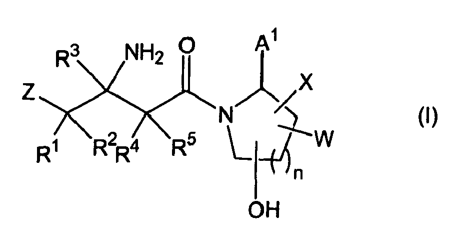 wherein the variables have the above described meanings, may be prepared using standard peptide coupling conditions, reagents and protective groups. For example, it may be possible to use 1-ethyl-3-(3-dimethylaminopropyl)carbodiimide hydrochloride (EDC) in combination with 1-hydroxybenzotriazole (HOBt) and a base (triethylamine or diisopropylethylamine) or *O*-(7-azabenzotriazol-1-yl)-N,N,N',N'-tetramethyluronium hexafluorophosphate (HATU) in the presence of a base, in solvents such as methylene chloride or *N,N*-dimethylformamide.

Scheme J outlines a procedure for using the amines formed according to Schemes A through F to synthesize compounds that are embodiments of the invention.

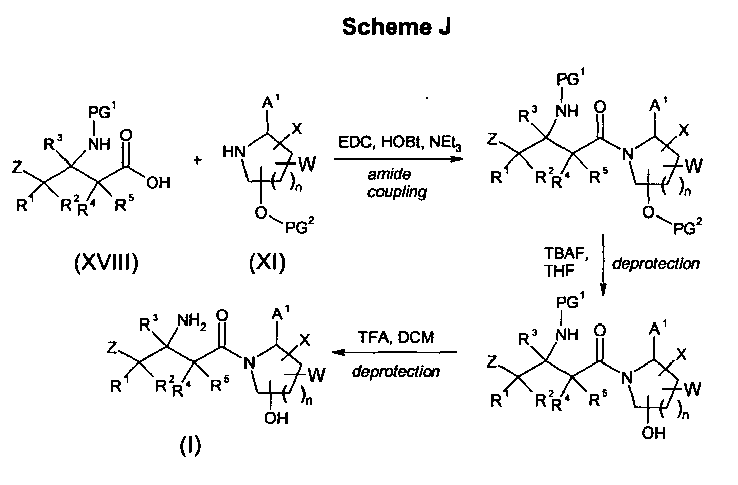

Silyl protecting groups for alcohols may be removed with tetra *n*-butyl ammonium fluoride (TBAF) solution in THF or by reaction with hydrogenfluoride in pyridine. The protective groups on the primary nitrogen may be removed with, for example, diethylamine in dichloromethane in the case of 9-fluorenylmethoxycarbonyl or using acidic conditions (such as trifluoroacetic acid in dichloro methane or hydrochloric acid in dioxane) in the case of *tert*-butoxycarbonyl, as described in Greene, Wuts Protective Groups in Organic Synthesis 3rd ed., Ed. Wiley-VCH, New York; 1999.

Accordingly, compounds with the general formula (II) 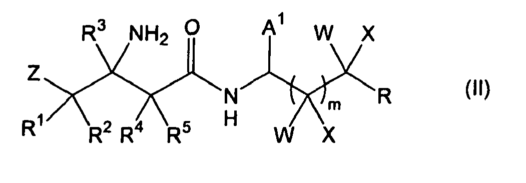 may be prepared by a similar procedure, as exemplified in Scheme K. In the first step amino building blocks (XVII) are coupled with beta amino acids (XVIII) to form the corresponding amides. Suitable reaction conditions are for example, the use of 1-ethyl-3-(3-dimethylaminopropyl)carbodiimide hydrochloride (EDC) in combination with 1-hydroxybenzotriazole (HOBt) and a base (triethylamine or diisopropylethylamine) or *O-*(7-azabenzotriazol-1-yl)-N,N,N',N'-tetramethyluronium hexafluorophosphate (HATU) in the presence of a base, in solvents such as methylene chloride or *N,N-*dimethylformamide. In the next steps the ester may be saponified with a hydroxide base, preferable lithium or sodium hydroxide in aqueous tetrahydrofurane, and the protecting group on the primary amine is removed. For example, diethylamine in dichloromethane in the case of 9-fluorenylmethoxycarbonyl or acidic conditions (such as trifluoroacetic acid in dichloromethane or hydrochloric acid in dioxane) in the case of *tert*-butoxycarbonyl can be employed, as described in Greene, Wuts Protective Groups in Organic Synthesis 3rd ed., Ed. Wiley-VCH, New York; 1999 to yield compounds (II) of the present invention.

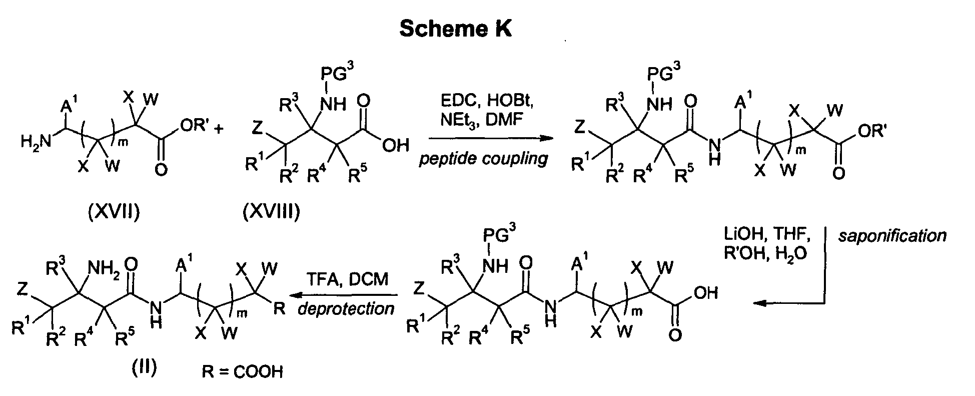

For the purification of intermediates or end products, flash chromatography on silica gel may be suitable for the free amines whereas the use of preparative HPLC leads to the isolation of the corresponding trifluoroacetic acid or formate salts.

### EXAMPLES

The following examples are provided so that the invention might be more fully understood. These examples are illustrative only and should not be construed as limiting the invention in any way.

### PREPARATIONS

### Example 1

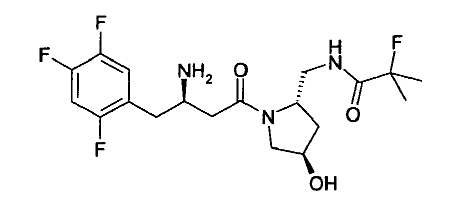

### Step 1

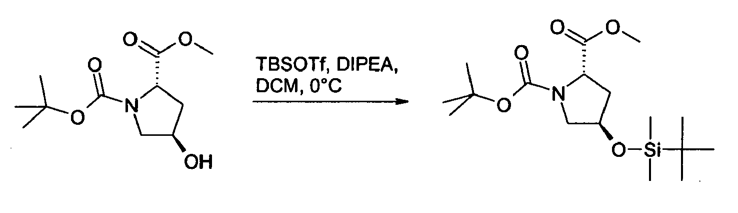

### (2S,4R)-4-(tert-Butyl-dimethyl-silanyloxy)-pyrrolidine-1,2-dicarboxylic acid 1-tert-butyl ester 2-methyl ester

930 mg (3.80 mmol, 1.00 eq) (2*S*,4*R*)-4-hydroxy-pyrrolidine-1,2-dicarboxylic acid 1*-tert-*butyl ester 2-methyl ester are dissolved in 38 mL of dichloromethane and the mixture is cooled with an ice bath to 0 °C. Then 2.19 mL (1.62 mg, 12.6 mmol, 3.30 eq) of diisopropylethylamine followed by 960 µL (1.11 g, 4.18 mmol, 1.10 eq) of *tert*-butyl dimethyl silyl triflate are added and stirring is continued for 1 h. To quench the reaction saturated sodium bicarbonate solution is added and the organic layer is separated and washed with water and brine. The organic layer is dried with sodium sulfate and the solvent is removed under reduced pressure. The product is purified by flash chromatography on silica gel with c-hexane:ethyl acetate as eluent.

¹H-NMR (300 MHz, d₆-DMSO) δ = 0.04 (s; 6H, 2xCH₃), 0.82 (s; 9H, 3xCH₃), 1.30-1.36 (d; 9H, 3xCH₃), 1.91-1.99 (m; 1H, CH), 2.07-2.09 (m; 1H, CH), 3.20-3.26 (m; 1H, CH), 3.41-3.44 (m; 1H. CH), 3.61-3.64 (d; 3H, CH₃), 4.17-4.20 (m; 1H. CH), 4.41 (s; 1H, CH).
LCMS (I) (5-95%, 5 min): rt 5.29; m/z 260 (M-boc), 301 (M-boc+ACN).

### Step 2

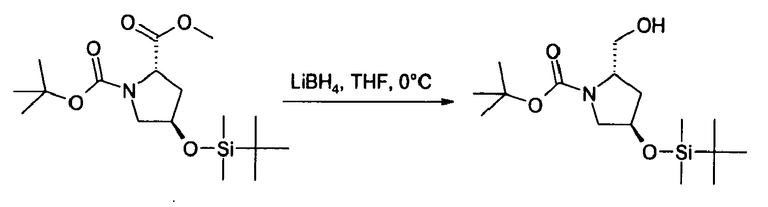

### (2S,4R)-4-(tert-Butyl-dimethyl-silanyloxy)-2-hydroxymethyl-pyrrolidine-1-carboxylic acid tert-butyl ester

1.00 g (2.67 mmol, 1.00 eq) of (2*S*,4*R*)-4-(*tert*-Butyl-dimethyl-silanyloxy)-pyrrolidine-1,2-dicarboxylic acid 1-*tert*-butyl ester 2-methyl ester are dissolved in 20 mL of tetrahydrofuran and cooled with an ice bath to 0 °C. Then 5.01 mL (10.0 mmol, 3.75 eq) of a 2.0 M solution of lithium borohydride in tetrahydrofuran are added and the resulting mixture is stirred for 5 h at 0 °C. Afterwards methanol is added and stirring is continued for 1 h at room temperature. The solvents are removed under reduced pressure, and the residue is digested with a 1:1 mixture of a saturated sodium bicarbonate solution and ethyl acetate. The organic layer is washed with water and brine, dried with sodium sulfate, and the solvent is removed under reduced pressure. The product does not require further purification and can be used in the next reaction steps.

¹H-NMR (300 MHz, d₆-DMSO) δ = 0.03 (s; 6H, 2xCH₃), 0.82 (s; 9H, 3xCH₃), 1.36 (s; 9H, 3xCH₃), 1.77-1.80 (m; 1H, CH), 1.91-2.00 (m; 1H, CH), 3.21-3.25 (m; 2H, CH₂), 3.25-3.45 (m; 2H, CH₂), 3.73-3.77 (m, 1 H, CH), 4.37-4.38 (m; 1 H, CH), 4.64-4.67 (t; 1 H, OH).
LCMS (I) (5-95%, 5 min): rt 4.97; m/z 232 (M-boc), 273 (M-boc+ACN), 276, 317 (M-Me).

### Steps 3 and 4

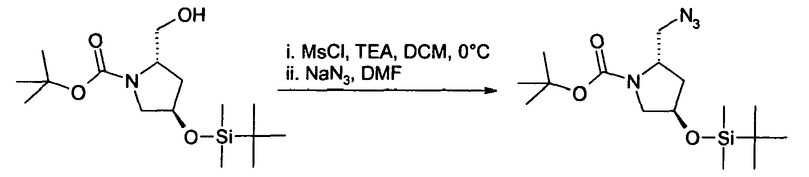

### (2S,4R)-2-Azidomethyl-4-(tert-butyl-dimethyl-silanyloxy)-pyrrolidine-1-carboxylic acid tert-butyl ester

950 mg (2.87 mmol, 1.00 eq) of (2*S*,4*R*)-4-(*tert*-butyl-dimethyl-silanyloxy)-2-hydroxymethyl-pyrrolidine-1-carboxylic acid *tert*-butyl ester are dissolved in 16 mL of dichloromethane, 959 µL (696 mg, 6.89 mmol, 2.40 eq) triethylamine is added and the solution is cooled with an ice bath to 0°C. Next, a solution of 266 µL (3.43 mmol, 1.20 eq) of methanesulfonyl chloride in 4 mL of dichloromethane is added dropwise and stirring is continued for 1 h at 0 °C. Afterwards the reaction mixture is washed with saturated sodium bicarbonate solution, water and brine, dried with sodium sulfate, and the solvent is removed under reduced pressure. The residue is dissolved in 20 mL of dimethylformamide and 279 mg (4.30 mmol, 1.50 eq) of sodium azide is added. The resulting mixture is heated for 3 h to 80 °C and the reaction progress is monitored by TLC (silica gel, eluent: c-hexane:ethyl acetate 90:10). After completion of the reaction the mixture is poured into brine and extracted thrice with ethyl acetate. The combined organic layers are washed with water and brine, dried over sodium sulfate, and the solvent is removed under reduced pressure. The crude product is purified by flash chromatography on silica gel with c-hexane:ethyl acetate as eluent.

¹H-NMR (300 MHz, d₆-DMSO) δ = 0.03 (s; 6H, 2xCH₃), 0.82 (s; 9H, 3xCH₃), 1.37 (s; 9H, 3xCH₃), 1.86 (m; 2.0, CH₂), 3.23-3.36 (m; 3H, CH and CH₂), 3.59-3.73 (m; 1H, CH), 3.93 (s; 1 H, CH), 4.37 (s; 1H, CH).
LCMS (I) (5-95%, 5 min): rt 5.71; m/z 257 (M-boc), 298 (M-boc+ACN), 329.

### Step 5

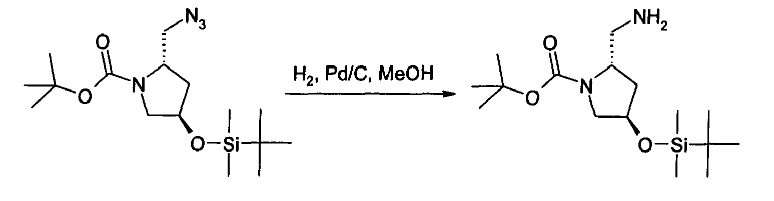

### (2S,4R)-2-Aminomethyl-4-(tert-butyl-dimethyl-silanyloxy)-pyrrolidine-1-carboxylic acid tert-butyl ester

651 mg (1.83 mmol, 1.00 eq) of (2*S*,4*R*)-2-azidomethyl-4-(tert-butyl-dimethyl-silanyloxy)-pyrrolidine-1-carboxylic acid *tert*-butyl ester are dissolved in 20 mL of methanol and 600 mL of 10 % palladium on charcoal is added. The reaction vessel is flushed with hydrogen and the reaction mixture is stirred under a hydrogen atmosphere for 1 h at room temperature. The palladium on charcoal is filtered off through celite and the filtrate is concentrated in vacuum. The crude product can be used in the next step without further purification.
¹H-NMR (300 MHz, d₆-DMSO) δ = 0.02 (s; 6H, 2xCH₃), 0.82 (s; 9H, 3xCH₃), 1.36 (s; 9H, 3xCH₃), 1.73-1.88 (m; 2H, 2xCH), 2.48-2.68 (m; 2H, 2xCH), 3.24-3.31 (m; 4H, NH₂ and CH₂), 3.69 (br s; 1H, CH), 4.33 (br s; 1 H, CH).
LCMS (I) (5-95%, 5 min): rt 3.12; m/z 231 (M-boc), 275, 316 (M-Me), 331 (M+H), 372 (M+H+ACN).

### Step 6

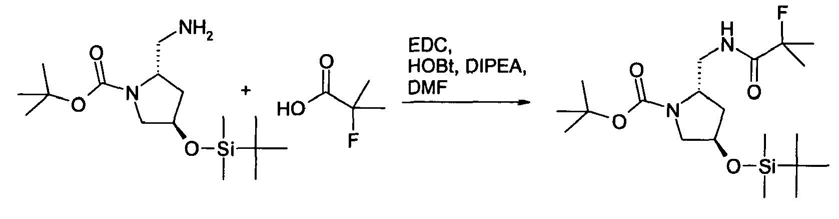

### (2S,4R)-4-(tert-Butyl-dimethyl-silanyloxy)-2-[(2-fluoro-2-methyl-propionylamino)-methyl]-pyrrolidine-1-carboxylic acid tert-butvl ester

219 mg (2.06 mmol, 2.20 eq) of 2-fluoro-2-methyl-propionic acid, 1.08 mL (800 mg, 6.19 mmol, 6.60 eq) of diisopropylethylamine, 418 mg (3.10 mmol, 3.30 eq) of HOBt and 467 mg (2.44 mmol, 2.60 eq) of EDC are subsequently dissolved in 5 mL of dimethylformamide. After stirring for 30 min at room temperature a solution of 310 mg (938 µmol, 1.00 eq) of (2*S*,4*R*)-2-aminomethyl-4-(tert-butyl-dimethyl-silanyloxy)-pyrrolidine-1-carboxylic acid *tert*-butyl ester in 5 mL dimethylformamide is added and stirring is continued overnight. Afterwards the reaction mixture is diluted with ethyl acetate and washed with brine, saturated sodium bicarbonate solution, water and brine. The organic layer is dried over sodium sulfate and the solvent is evaporated. The crude product is purified by flash chromatography on silica gel using c-hexane:ethyl acetate as eluent.

¹H-NMR (300 MHz, d₆-DMSO) δ = 0.02 (s; 6H, 2xCH₃), 0.81 (s; 9H, 3xCH₃), 1.38-1.40 (m; 12H, 4xCH₃), 1.45 (s; 3H, CH₃), 1.76-1.77 (m; 2H, CH₂), 3.20-3.26 (m; 4H, 2xCH₂), 3.87-3.98 (m; 1H, CH), 4.34 (s; 1 H, CH), 8.12 (br m; 1 H, NH).
LCMS (I) (5-95%, 5 min): rt 5.30; m/z 319 (M-boc), 360 (M-boc+ACN), 419 (M+H).

### Step 7

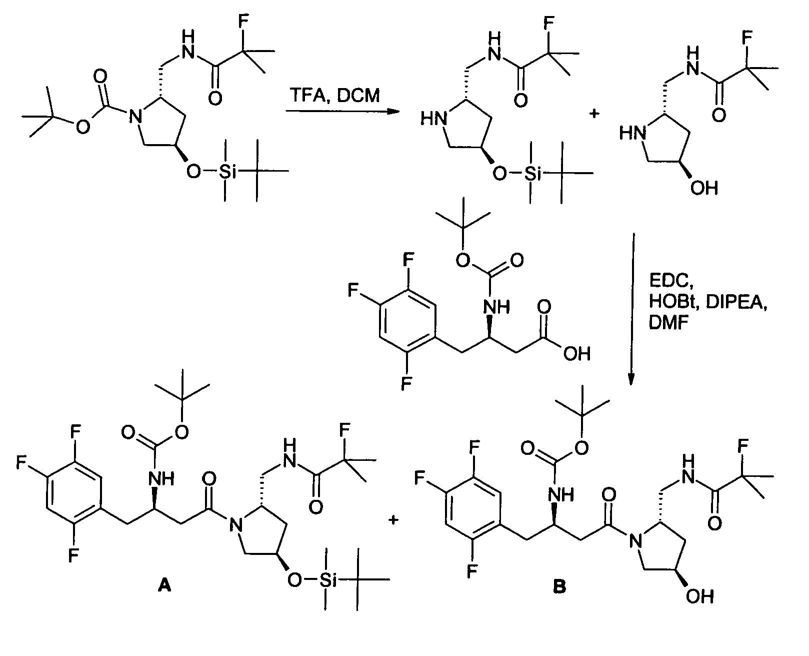

### [(R)-3-{(2S,4R)-4-(tert-Butyl-dimethyl-silanyloxy)-2-[(2-fluoro-2-methyl-propionylamino)-methyl]-pyrrolidin-1-yl}-3-oxo-1-(2,4,5-trifluoro-benzyl)-propyl]-carbamic acid tert-butyl ester A and [(R)-3-{(2S,4R)-2-[(2-Fluoro-2-methyl-propionylamino)-methyll-4-hydroxy-pyrrolidin-1-yl}-3-oxo-1-(2,4,5-trifluoro-benzyl)-propyl]-carbamic acid tert-butyl ester B

310 mg (741 µmol, 1.00 eq) of (2*S*,4*R*)-4-(*tert*-butyl-dimethyl-silanyloxy)-2-[(2-fluoro-2-methyl-propionylamino)-methyl]-pyrrolidine-1-carboxylic acid *tert*-butyl ester are dissolved in 6 mL of dichloromethane. 3 mL of trifluoroacetic acid are added and the reaction mixture is stirred at room temperature for 1 h. Afterwards the solvent is removed under reduced pressure and the crude product is dissolved in 4 mL of dimethylformamide. This solution is added to 272 mg (815 µmol, 1.10 eq) of (*R*)-3-*tert-*butoxycarbonylamino-4-(2,4,5-trifluoro-phenyl)-butyric acid in 4 mL of dimethylformamide that had been treated for 30 min with 650 µL (478 mg, 3.70 mmol, 5.00 eq) of diisopropylethylamine, 248 mg (1.83 mmol, 1.50 eq) hydroxybenzotriazole and 234 mg (1.22 mmol, 1.50 eq) of EDC. The resulting mixture is stirred over night at room temperature, diluted with ethyl acetate and washed with brine, saturated sodium bicarbonate solution, water and brine. After drying over sodium sulfate the solvent is removed under reduced pressure and the crude products are separated by flash chromatography with dichloromethane:methanol (gradient 100:0 to 90:10) as eluent.
**A** LCMS (I) (5-95%, 5 min): rt 5.33; m/z 534 (M-boc), 578, 634 (M+H), 656 (M+Na).
**B** LCMS (I) (5-95%, 5 min): rt 3.59; m/z 420 (M-boc), 464, 520 (M+H), 542 (M+Na).

### Step 8

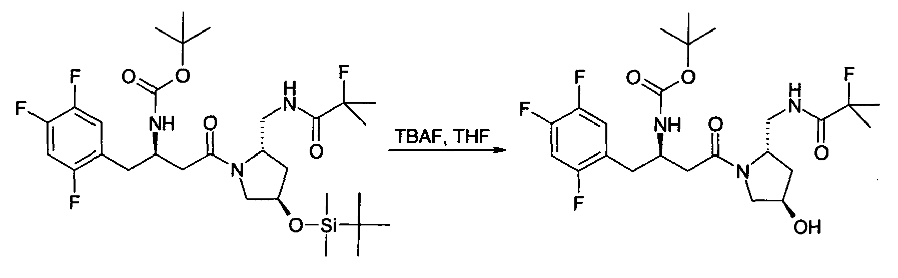

### [(R)-3-{(2S.4R)-4-(tert-Butyl-dimethyl-silanyloxy)-2-[(2-fluoro-2-methyl-propionylamino)-methyl]-pyrrolidin-1-yl}-3-oxo-1-(2,4,5-trifluoro-benzyl)-propyl]-carbamic acid tert-butyl ester

120 mg (189 µmol, 1.00 eq) [(*R*)-3-{(2*S*,4*R*)-2-[(2-Fluoro-2-methyl-propionylamino)-methy)]-4-hydroxy-pyrrolidin-1-yl}-3-oxo-1-(2,4,5-trifluoro-benzyl)-propyl]-carbamic acid *tert-butyl* ester is dissolved in 2 mL of tetrahydrofuran and the solution is cooled with an ice bath to 0 °C. Then a 1.0 M solution of tetra-n-butylammonium fluorid in tetrahydrofuran is added and stirring is continued at 0 °C for 90 min. Afterwards the reaction is quenched by the addition of a saturated sodium bicarbonate solution and the organic layer is diluted with ethyl acetate and washed with water and brine. The organic layer is next dried with sodium sulfate and concentrated under vacuum. The crude product is used in the next step without further purification.

LCMS (I) (5-95%, 5 min): rt 3.59; m/z 420 (M-boc), 464, 520 (M+H), 542 (M+Na).

### Step 9

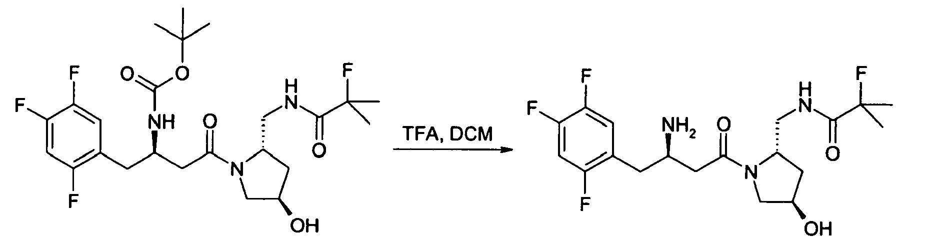

### N-{(2S,4R)-1-[(R)-3-Amino-4-(2,4,5-trifluoro-phenyl)-butvryl]-4-hydroxy-pyrrolidin-2-ylmethyl}-2-fluoro-2-methyl-propionamide

193 mg (371 µmol, 1.00 eq) of [(*R*)-3-{(2*S*,4*R*)-2-[(2-fluoro-2-methyl-propionylamino)-methyl]-4-hydroxy-pyrrolidin-1-yl}-3-oxo-1-(2,4,5-trifluoro-benzyl)-propyl]-carbamic acid *tert-*butyl ester are dissolved in 5 mL of dichloromethane and 2 mL of trifluoroacetic acid are added. The resulting mixture is stirred for 2 h at room temperature. Afterwards the solvents are removed at reduced pressure, and the crude product is taken up in a mixture of 4 mL of methanol and 2 mL of aqueous ammonia solution in water. The solvents are removed under reduced pressure, and the crude product is purified by preparative HPLC.

¹H-NMR (300 MHz, d₆-DMSO) δ = 1.36-1.42 (m; 6H, 2xCH₃), 1.76-2.08 (m; 2H, 2xCH), 2.39-2.61 (m; 2H, 2xCH), 2.80 (s; 2H, CH₂), 3.14-3.55 (m; 5H, CH and 2xCH₂), 4.12-4.27 (m; 2H, CH₂), 7.45-7.54 (m; 2H, aryl-H), 7.45-7.54 (m; 2H, aryl-H), 8.17-8.23 (m; 2H, OH, NH).
LCMS (II) (1-30%, 10 min): rt 6.17; m/z 420 (M+H), 442 (M+Na).

### Example 2

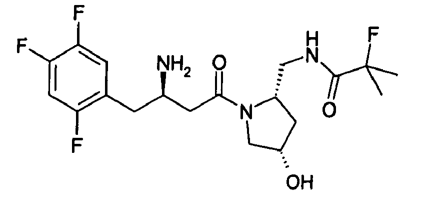

### Step 1

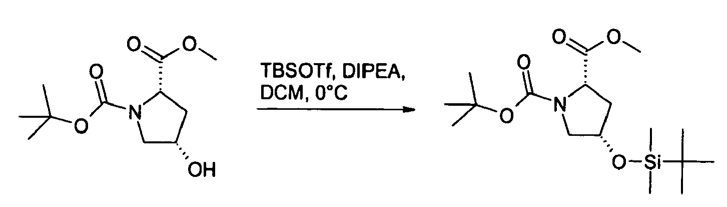

### (2S,4S)-4-(tert-Butyl-dimethyl-silanyloxy)-pyrrolidine-1,2-dicarboxylic acid 1-tert-butyl ester 2-methyl ester

The title compound can be prepared according to example 1, step 1 from (2*S*,4*S*)-4-hydroxy-pyrrolidine-1,2-dicarboxylic acid 1-*tert*-butyl ester 2-methyl ester.

LCMS (I) (5-95%, 5 min): rt 5.19; m/z 260 (M-boc), 301 (M-boc+ACN), 304, 345 (M-Me).

### Step 2

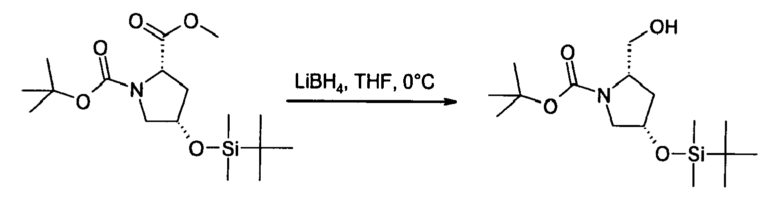

### (2S,4S)-4-(tert-Butyl-dimethyl-silanyloxy)-2-hydroxymethyl-pvrrolidine-1-carboxylic acid tert-butyl ester

The title compound can be prepared according to example 1, step 2 from (2*S*,4*S*)-4-(*tert*-butyl-dimethyl-silanyloxy)-pyrrolidine-1,2-dicarboxylic acid 1-*tert*-butyl ester 2-methyl ester.

LCMS (I) (5-95%, 5 min): rt 4.90; m/z 232 (M-boc), 273 (M-boc+ACN), 276, 317 (M-Me).

### Steps 3 and 4

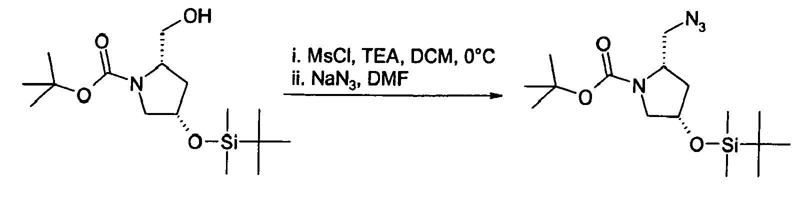

### (2S,4S)-2-Azidomethyl-4-(tert-butyl-dimethyl-silanyloxy)-pyrrolidine-1-carboxylic acid tert-butyl ester

The title compound can be prepared according to example 1, steps 3 and 4 from (2*S*,4*S*)-4-(*tert*-butyl-dimethyl-silanyloxy)-2-hydroxymethyl-pyrrolidine-1-carboxylic acid *tert-*butyl ester.
¹H-NMR (300 MHz, d₆-DMSO) δ = 0.04 (s; 6H, 2xCH₃), 0.84 (s; 9H, 3xCH₃), 1.39 (s; 9H, 3xCH₃), 1.71-1.73 (m; 1H, CH), 2.11-2.13 (m; 1H, CH), 3.00-3.05 (m; 1H, CH), 3.42-3.53 (m; 3H, CH₂, CH), 3.85 (m; 1 H, CH), 4.37 (m; 1H, CH).
LCMS (I) (5-95%, 5 min): rt 5.70; m/z 257 (M-boc), 298, 303, 342 (M-Me).

### Step 5

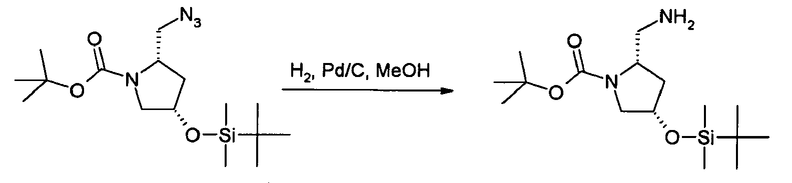

### (2S,4S)-2-Aminomethyl-4-(tert-butyl-dimethyl-silanyloxy)-pyrrolidine-1-carboxylic acid tert-butyl ester

The title compound can be prepared according to example 1, step 5 from (2*S*,4*S*)-2-azidomethyl-4-(tert-butyl-dimethyl-silanyloxy)-pyrrolidine-1-carboxylic acid *tert-*butyl ester.
LCMS (I) (5-95%, 5 min): rt 3.04; m/z 231 (M-boc), 275, 316 (M-Me), 331 (M+H), 372 (M+H+ACN).

### Step 6

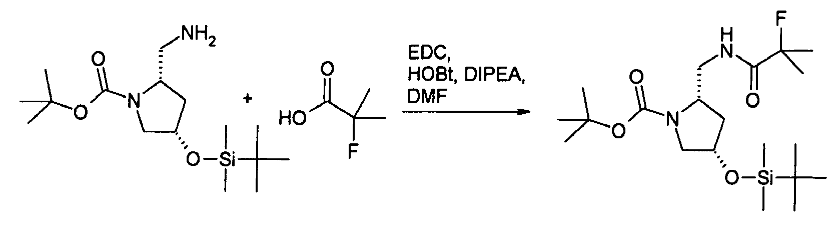

### (2S,4R)-4-(tert-Butyl-dimethyl-silanyloxy)-2-[2-fluoro-2-methyl-propionylamino-methyl]-pyrrolidine-1-carboxylic acid tert-butyl ester

The title compound can be prepared according to example 1, step 6 from (2S,4S)-2-aminomethyl-4-(tert-butyl-dimethyl-silanyloxy)-pyrrolidine-1-carboxylic acid *tert*-butyl ester.
LCMS (I) (5-95%, 5 min): rt 2.74; m/z 319 (M-boc), 360 (M-boc+ACN).

### Step 7

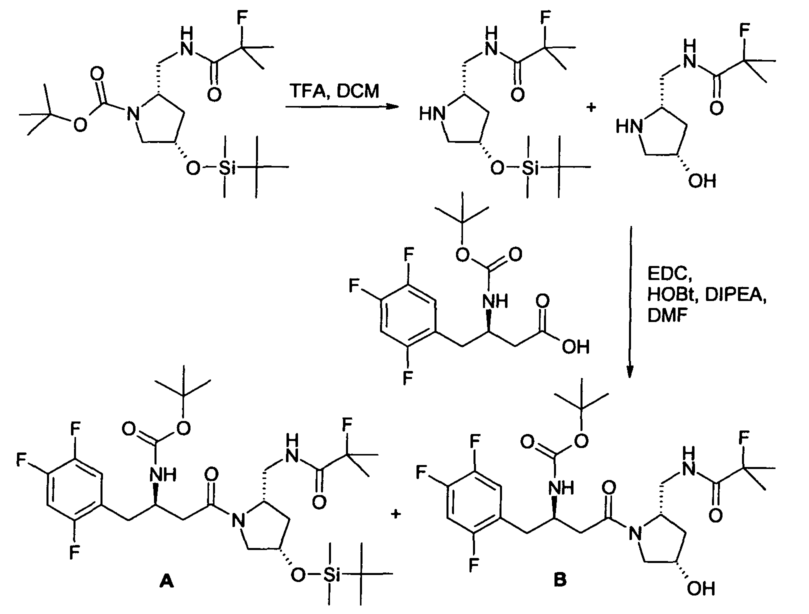

### [(R)-3-{(2S,4R)-4-(tert-Butyl-dimethyl-silanyloxy)-2-[(2-fluoro-2-methyl-propionylamino)-methyl]-pyrrolidin-1-yl}-3-oxo-1-(2,4,5-trifluoro-benzyl)-propyl]-carbamic acid tert-butyl ester A and [(R)-3-{(2S,4S)-2-[(2-Fluoro-2-methyl-propionylamino)-methyl]-4-hydroxy-pyrrolidin-1-yl}-3-oxo-1-(2,4,5-trifluoro-benzyl)-propyl]-carbamic acid tert-butyl ester B

The title compound can be prepared according to example 1, step 7 from (2S,4R)-4-(*tert*-Butyl-dimethyl-silanyloxy)-2-[(2-fluoro-2-methyl-propionylamino)-methyl)-pyrrolidine-1-carboxylic acid *tert*-butyl ester.
**A** LCMS (I) (5-95%, 5 min): rt 5.23; m/z 534 (M-boc), 634 (M+H), 656 (M+Na).
**B** LCMS (I) (5-95%, 5 min): rt 3.43; m/z 420 (M-boc), 520 (M+H), 542 (M+Na).

### Step 8

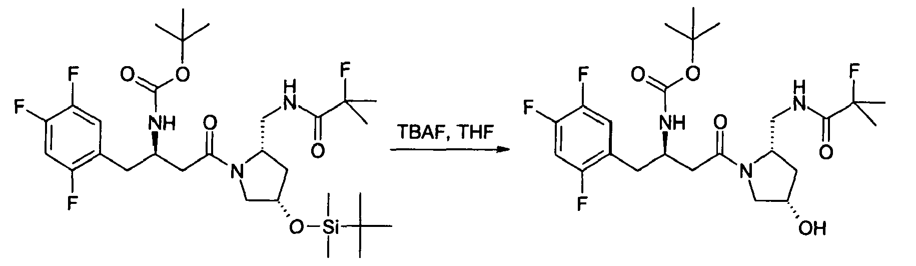

### [(R)-3-{(2S,4R)-4-(tert-Butyl-dimethyl-silanyloxy)-2-[(2-fluoro-2-methyl-propionylamino)-methyl]-pyrrolidin-1-yl}-3-oxo-1-(2,4,5-trifluoro-benzyl)-propyl]-carbamic acid tert-butyl ester

The title compound can be prepared according to example 1, step 7 from [(*R*)-3-{(2*S*,4*S*)-2-[(2-fluoro-2-methyl-propionylamino)-methyl]-4-hydroxy-pyrrolidin-1-yl}-3-oxo-1-(2,4,5-trifluoro-benzyl)-propyl]-carbamic acid *tert*-butyl ester.

LCMS (I) (5-95%, 5 min): rt 3.43; m/z 420 (M-boc), 520 (M+H), 542 (M+Na).

### Step 9

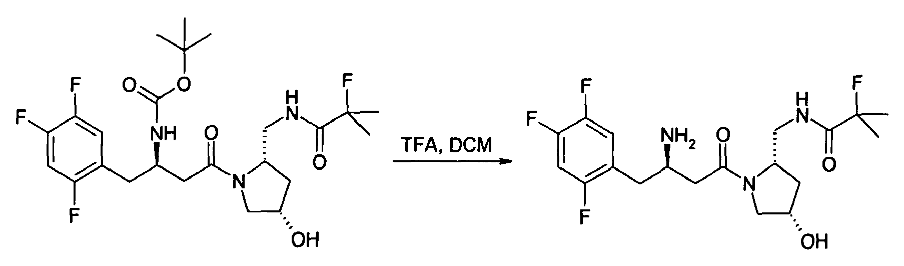

### N-{(2S,4S)-1-[(R)-3-Amino-4-(2,4,5-trifluoro-phenyl)-butyryl]-4-hydroxy-pvrrolidin-2-ylmethyl}-2-fluoro-2-methyl-propionamide

The title compound can be prepared according to example 1, step 9 from [(*R*)-3-{(2*S*,4*S*)-2-[(2-fluoro-2-methyl-propionylamino)-methyl]-4-hydroxy-pyrrolidin-1-yl}-3-oxo-1-(2,4,5-trifluoro-benzyl)-propyl]-carbamic acid *tert*-butyl ester or step 8b from [(*R*)-3-{(2*S*,4*R*)-4-(*tert*-buty-dimethyl-silanyloxy)-2-[(2-fluoro-2-methyl-propionylamino)-methyl]-pyrrolidin-1-yl}-3-oxo-1-(2,4,5-trifluoro-benzyl)-propyl]-carbamic acid *tert*-butyl ester.

¹H-NMR (300 MHz, d₆-DMSO) δ = 1.35-1.47 (m; 6H, 2xCH₃), 1.64-1.86 (m; 1H, CH), 1.92-2.10 (m; 1 H, CH), 2.39-2.61 (m; 2H, CH₂), 2.76-2.77 (m; 2H, CH₂), 3.11-3.56 (m; 7H, NH₂, OH, CH₂ and 2xCH), 4.17-4.25 (m; 2H, CH₂), 7.45-7.50 (m; 2H, aryl-H).
LCMS (II) (1-30%, 10 min): rt = 6.60 min; m/z 420 [M+H], 442 [M+Na].

### Example 3

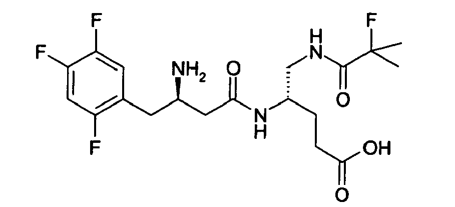

### Step 1

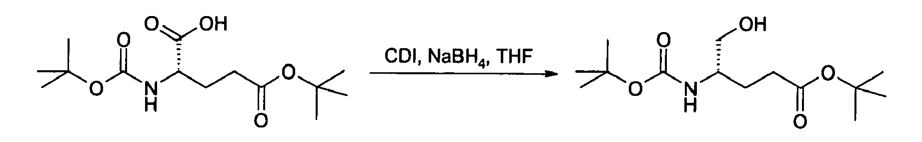

### (S)-4-tert-Butoxycarbonylamino-5-hydroxy-pentanoic acid tert-butyl ester

800 mg (2.64 mmol, 1.00 eq) of (*S*)-2-*tert*-butoxycarbonylamino-pentanedionic acid 5-*tert*-butyl ester are dissolved in 25 mL of tetrahydrofuran and 641 mg (3.96 mmol, 1.50 eq) CDI are added. The resulting solution is stirred for 1 h at room temperature and 150 mg (3.96 mmol, 1.50 eq) of sodiumborohydride is added. The reaction mixture is stirred for 1 h at room temperature, quenched with acetone and evaporated to dryness. The crude product is dissolved in dichloromethane, washed with saturated bicarbonate solution, water and brine. The organic layer is dried over sodium sulfate and concentrated in vacuum. The product is used in the following step without further purification.

LCMS (I) (5-95%, 5 min): rt 3.49; m/z 219, 290, 312, 353.

### Steps 2 and 3

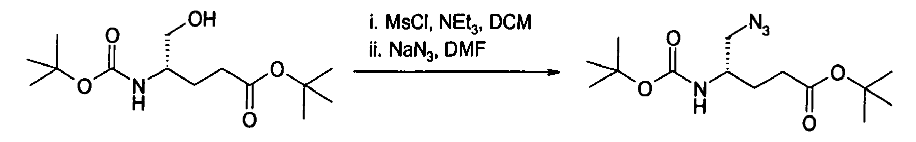

### (S)-5-Azido-4-tert-butoxycarbonylamino-pentanoic acid tert-butyl ester

758 mg (2.62 mmol, 1.00 eq) of (*S*)-4-*tert*-butoxycarbonylamino-5-hydroxy-pentanoic acid *tert*-butyl ester are dissolved in 20 mL of dichloromethane and cooled with an ice bath. Then 876 µL (636 mg, 6.29 mmol, 2.40 eq) of triethylamine followed by 243 µL (360 mg, 3.14 mmol, 1.20 eq) methanesulfonyl chloride in 5 mL of dichloromethane are added to the reaction mixture. The resulting solution is stirred for 1 h at 0°C, washed with saturated sodium bicarbonate solution, water and brine, dried with sodium sulfate and evaporated to dryness. The crude product is dissolved in 25 mL of dimethylformamide and 255 mg (3.93 mmol, 1.50 eq) of sodium azide are added. The reaction mixture is heated to 80°C overnight, diluted with 100 mL of ethyl acetate and washed with brine, saturated sodium bicarbonate solution, water and brine. Afterwards the solution is dried with sodium sulfate, the solvent is removed under reduced pressure and the crude product is purified by flash chromatography on silica gel using c-hexane:ethyl acetat as eluent.

LCMS (I) (5-95%, 5 min): rt 4.32; m/z 200, 203, 244, 378 (M+Na+ACN).

### Step 4

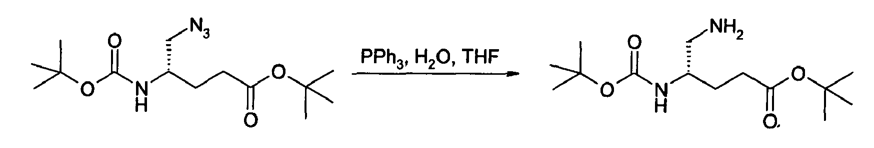

### (S)-5-Amino-4-tert-butoxycarbonylamino-pentanoic acid tert-butyl ester

347 mg (1.10 mmol, 1.00 eq) of (*S*)-5-azido-4-*tert*-butoxycarbonylamino-pentanoic acid *tert*-butyl ester are dissolved in 11 mL of tetrahydrofuran, treated with 347 mg (1.32 mmol, 1.20 eq) of triphenylphosphane and the reaction mixture is heated to reflux for 3 h. Then 1 mL of water is added to the solution and stirring is continued for 1 h at room temperature. The solvents are evaporated to dryness and the crude product is purified by flash chromatography on silica gel using dichloromethane:methanol (containing 5 % of aqueous ammonia) as eluent.

LCMS (I) (5-95%, 5 min): rt 2.31; m/z 233, 289 (M+H).

### Step 5

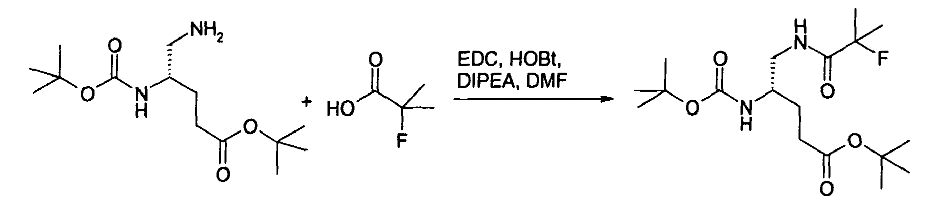

### (S)-4-tert-Butoxycarbonylamino-5-(2-fluoro-2-methyl-propionylamino)-pentanoic acid tert-butyl ester

117 mg (1.10 mmol, 2.20 eq) of 2-fluoro-2-methyl-propionic acid is dissolved in 4 mL of dimethylformamide and 0.58 mL (425 mg, 3.30 mmol, 6.60 eq) of diisopropylethylamine, 223 mg (1.65 mmol, 3.30 eq) of hydroxybenzotriazole and 249 mg (1.30 mmol, 2.60 eq) of EDC are added, and the resulting solution is stirred for 30 min at room temperature. Then a solution of 144 mg (0.50 mmol, 1.00 eq) of (S)-5-amino-4-*tert*-butoxycarbonylamino-pentanoic acid *tert*-butyl ester in 4 mL of dimethylformamide is added and stirring is continued at room temperature overnight. The solution is diluted with ethyl acetate, washed with brine, saturated sodium bicarbonate solution, water and brine, and dried with sodium sulfate. The solvent is removed under reduced pressure and the crude product is purified by flash chromatography on silica gel using ethyl acetate:c-hexane as eluent.

LCMS (I) (5-95%, 5 min): rt 3.96; m/z 277 (M-boc), 377 (M+H), 399 (M+Na), 440 (M+Na+ACN).

### Step 6 and 7

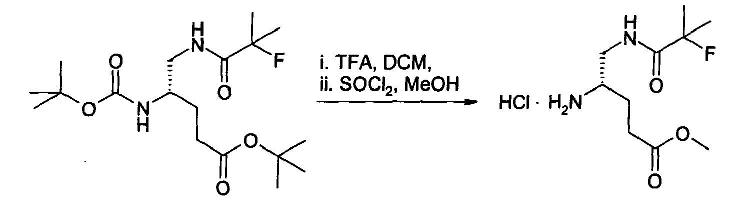

### (S)-4-Amino-5-(2-fluoro-2-methyl-propionylamino)-pentanoic acid methyl ester hydrochloride

88 mg (234 µmol, 1.00 eq) of (*S*)-4-*tert*-butoxycarbonylamino-5-(2-fluoro-2-methy)-propionylamino)-pentanoic acid *tert*-butyl ester are dissolved in 2 mL of dichloromethane, and are treated with 1 mL of trifluoroacetic acid. Stirring is continued for 2 h, the solvents are removed under reduced pressure and the crude product is dissolved in 2 mL of methanol. Then 37.5 µL (514 µmol, 2.20 eq) thionylchloride are added and the reaction mixture is stirred over night. The solvent is removed under vacuum and the crude product is used in the next step without further purification.

¹H-NMR (300 MHz, d₆-DMSO) δ = 1.41-1.49 (m; 6H, 2xCH₃), 1.73-1.76 (m; 3H, CH₂, CH), 2.02-2.06 (m; 1 H, CH), 3.04-3.22 (m; 6H, CH₃, CH₂, CH), 8.00 (br s; 3H, NH₃), 8.30 (br s; 1 H, NH).

LCMS (I) (5-95%, 5 min): rt 0.88; m/z 235 (M+H).

### Step 8

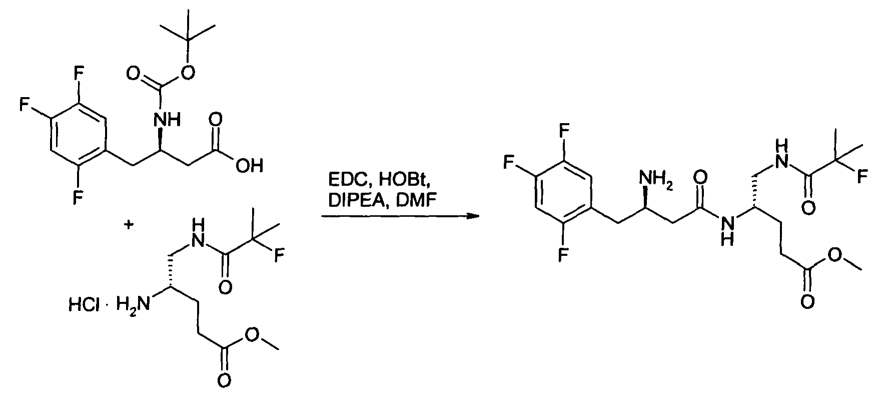

### (S)-4-[(R)-3-tert-Butoxycarbonylamino-4-(2,4,5-trifluoro-phenyl)-butyrylaminol-5-(2-fluoro-2-methyl-propionylamino)-pentanoic acid methyl ester

70 mg (209 µmol, 1.10 eq) of (*R*)-3-*tert*-butoxycarbonylamino-4-(2,4,5-trifluoro-phenyl)-butyric acid are dissolved in 2 mL of dimethylformamide. 170 µL (123 mg, 948 µmol, 5.00 eq) of diisopropylethylamine, 63 mg (469 µmol, 1.50 eq) hydroxybenzotriazole and 60 mg (313 µmol ,1.50 eq) EDC are added and the resulting mixture is stirred for 30 min at room temperature. Then a solution of 79 mg (190 µmol, 1.00 eq) of (*S*)-4-amino-5-(2-fluoro-2-methyl-propionylamino)-pentanoic acid methyl ester hydrochloride in 2 mL of dimethylformamide is added and the reaction mixture is stirred at room temperature overnight. The solution is diluted with ethyl acetate, washed with brine, saturated sodium bicarbonate solution, water and brine, and dried with sodium sulfate. The solvent is removed under reduced pressure and the crude product is purified by flash chromatography on silica gel using ethyl acetate:*c-*hexane as eluent.

¹H-NMR (300 MHz, d₆-DMSO) δ = 1.17-1.24 (m; 9H, 3xCH₃), 1.37-1.43 (m; 7H, 2xCH₃, CH), 1.55-1.71 (m; 1 H, CH), 2.09-2.31 (m; 4H, 2xCH₂), 2.78-2.82 (m; 2H, CH₂), 3.08-3.10 (m; 2H, CH₂), 3.84-3.96 (m; 2H, 2xCH), 6.66 (d; 1H, NH), 7.24-7.26 (m; 1 H, aryl-H), 7.42-7.44 (m; 1H, aryl-H), 7.55 (d; 1H, NH), 7.97 (br s; 1H, NH), 12.0 (br s; 1H, COOH).
LCMS (I) (5-95%, 5 min): rt 3.86; m/z 450(M-boc), 494, 550 (M+H), 572 (M+Na).

### Step 9

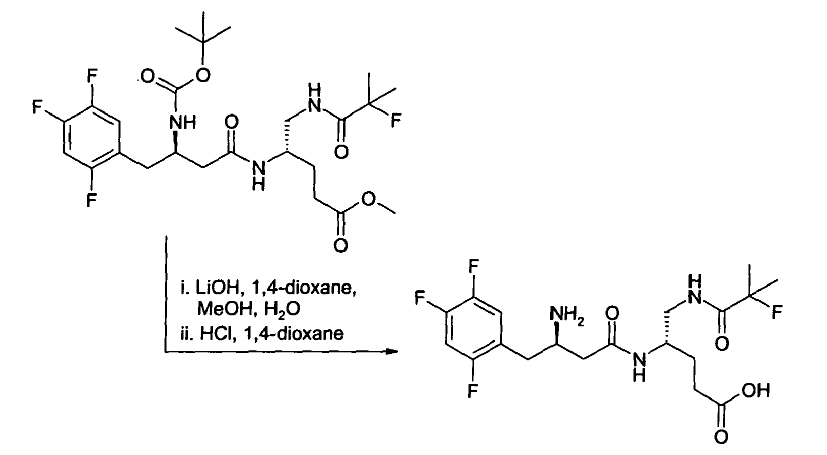

### (S)-4-[(R)-3-Amino-4-(2,4,5-trifluoro-phenyl)-butyrylamino]-5-(2-fluoro-2-methyl-propionylamino)-pentanoic acid

104 mg (189 µmol, 1.00 eq) of (*S*)-4-[(*R*)*-*3*-tert*-butoxycarbonylamino-4-(2,4,5-trifluorophenyl)-butyrylamino]-5-(2-fluoro-2-methyl-propionylamino)-pentanoic acid methyl ester are dissolved in 5 ml of a hot 1:1 mixture of 1,4-dioxane and methanol. Then a solution of 135 mg (378 µmol, 1.00 eq) of lithium hydroxide mono hydrate in 2mL of water. The reaction mixture is stirred for 2 h at room temperature. After monitoring by TLC (silica gel, c-hexane:ethyl acetate 30:70) shows complete conversion, the solvent is removed under reduced pressure, and the crude product is treated with an aqueous 0.1 M HCl solution. The insoluble material is filtered off, and the residue is treated with hot methanol. The acid dissolves and the remaining insoluble material is filtered off. The filtrate containing the desired intermediate is concentrated under reduced pressure. The residue is redissolved in 5 mL 1,4-dioxane, treated with 5 mL of a 2.0 M solution of HCl in 1,4-dioxane and stirring is continued at room temperature overnight. The solvent is removed under reduced pressure and the final product is purified by preparative HPLC.

¹H-NMR (formic acid salt, 300 MHz, d₆-DMSO) δ = 1.35-1.42 (m; 6H, 2xCH₃), 1.42-1.46 (m; 1 H, CH), 1.63-1.65 (m; 1 H, CH), 2.04-2.17 (m; 4H, 2xCH₂), 2.53-2.65 (m; 3H, CH, CH₂), 3.07-3.11 (m; 4H, NH₂, CH₂), 3.83-3.84 (m; 1 H, CH), 7.40-7.47 (m; 2H, aryl-H), 7.67-7.70 (m; 1 H, NH), 7.97 (br s; 1 H, NH).
LCMS (II) (10-60%, 10 min): rt = 3.67 min; m/z 436 [M+H], 458 [M+Na].

### ASSAYS

Inhibition of DPP-IV peptidase activity was monitored with a continuous fluorimetric assay. This assay is based on the cleavage of the substrate Gly-Pro-AMC (Bachem) by DPP-IV, releasing free AMC. The assay is carried out in 96-well microtiterplates. In a total volume of 100 µl, compounds are preincubated with 50 pM DPP-IV employing a buffer containing 10mM Hepes, 150mM NaCl, 0.005% Tween 20 (pH 7.4). The reaction is started by the addition of 16 µM substrate and the fluorescence of liberated AMC is detected for 10 minutes at 25 °C with a fluorescence reader (BMG-Fluostar; BMG-Technologies) using an excitation wavelength of 370 nm and an emission wavelength of 450 nm. The final concentration of DMSO is 1 %. The inhibitory potential of the compounds were determined. DPP-IV activity assays were carried out with human and porcine DPP-IV (see below); both enzymes showed comparable activities.

Soluble human DPP-IV lacking the transmembrane anchor (GIy31-Pro766) was expressed in a recombinant YEAST-strain as Pre-Pro-alpha-mating fusion. The secreted product (rhuDPP-IV-Gly31-Pro766) was purified from fermentation broth (>90% purity).

The examples 1-3 show a % inhibition of about 2 µM or less.

The compounds of the present invention also exhibit excellent physicochemical properties that are pharmacologically relevant such as aqueous solubility, plasma protein binding, metabolic stability and the like. Due to these properties, the claimed compounds show improved pharmacokinetic properties such as good absorption in the gastrointestinal tract, low first-pass metabolism and the like.

## Claims

1. A compound of formula (I) 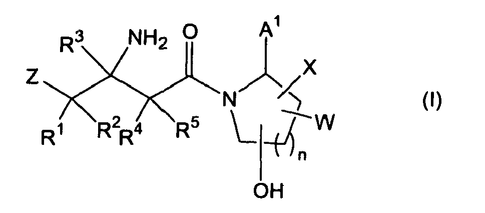 or a pharmaceutically acceptable salt thereof, wherein
Z is selected from the group consisting of
phenyl;
naphthyl;
C₃₋₇ cycloalkyl;
heterocycle; and
heterobicycle;
wherein Z is optionally substituted with one, or independently from each other, more of
halogen;
CN;
OH;
=O, where the ring is at least partially saturated;
C₁₋₆ alkyl, optionally substituted with one or more F; and
O-C₁₋₆ alkyl, optionally substituted with one or more F;
R¹, R², R⁴ and R⁵ are independently from each other selected from the group consisting of
H;
F;
OH;
C₁₋₆ alkyl, optionally substituted with one or more F; and
O-C₁₋₆ alkyl, optionally substituted with one or more F;
and/or R¹ and R² optionally form together C₃₋₇ cycloalkyl, which is optionally substituted with one or more F;
and/or R² and R³ optionally form together C₃₋₇ cycloalkyl, which is optionally substituted with one or more F;
and/or R³ and R⁴ optionally form together C₃₋₇ cycloalkyl, which is optionally substituted with one or more F;
and/or R⁴ and R⁵ optionally form together C₃₋₇ cycloalkyl, which is optionally substituted with one or more F;
R³ is H or C₁₋₆ alkyl;
n is 0, 1 or 2;
X is
H;
F;
CH₃;
OCH₃;
OH;
W is
H;
F;
A¹ is
-C(R⁶R⁷)-Y-T;
R⁶ and R⁷ are independently from each other selected from the group consisting of
H;
F; and
C₁₋₆ alkyl, optionally substituted with one or more F;
or R⁶ and R⁷ optionally form together C₃₋₇ cycloalkyl, which is optionally substituted with one or more F;
Y is selected from the group consisting of
-O-;
-C₁₋₆ alkyl-O-;
-N(R⁸)-;
-C₁₋₆ alkyl-N(R⁸)-_{;}
-S-;
-C₁₋₆ alkyl-S-;
-S(O)-;
-C₁₋₆ alkyl-S(O)-;
-S(O)₂-; and
-C₁₋₆ alkyl-S(O)₂-;
wherein each C₁₋₆ alkyl is optionally substituted with one or more F;
R⁸ and T are independently from each other T¹-T² or T²;
T¹ is selected from the group consisting of
-C₁₋₆ alkyl-;
-C₁₋₆ alkyl-O-;
-C₁₋₆ alkyl-N(R⁹)-;
-C(O)-;
-C(O)-C₁₋₆ alkyl-;
-C(O)-C₁₋₆ alkyl-O-;
-C(O)-C₁₋₆ alkyl-N(R⁹)-;
-C(O)O-;
-C(O)O-C₁₋₆ alkyl-;
-C(O)O-C₁₋₆ alkyl-O-;
-C(O)O-C₁₋₆ alkyl-N(R⁹)-;
-C(O)N(R⁹)-;
-C(O)N(R⁹)-C₁₋₆ alkyl-;
-C(O)N(R⁹)-C₁₋₆ alkyl-O-;
-C(O)N(R⁹)-C₁₋₆ alkyl-N(R¹⁰)-;
-S(O)₂-;
-5(O)₂-C₁₋₆ alkyl-;
-S(O)₂-C₁₋₆ alkyl-O-; and
-S(O)₂-C₁₋₆ alkyl-N(R⁹)-;
wherein each C₁₋₆ alkyl is optionally substituted with one or more F;
R⁹ and R¹⁰ are independently from each other H or C₁₋₆ alkyl, optionally substituted with one or more F;
T² is selected from the group consisting of
H;
F;
CF₃;
C₁₋₆ alkyl, optionally substituted with one or more halogen atoms;
phenyl;
naphthyl;
wherein phenyl and naphthyl are optionally substituted with one, or independently from each other, more of
halogen;
CN;
R¹¹;
COOH;
OH;
C(O)NH₂;
S(O)₂NH₂;
COOT³;
OT³;
C(O)NHT³;
S(O)₂NHT³; or
T³;
C₃₋₇ cycloalkyl;
heterocycle; and
heterobicycle;
wherein C₃₋₇ cycloalkyl, heterocycle and heterobicycle are optionally substituted with one, or independently from each other, more of halogen;
CN;
R¹²;
OH;
=O, where the ring is at least partially saturated;
NH₂;
COOH;
C(O)NH₂;
S(O)₂NH₂;
COOT³;
OT³;
C(O)NHT³;
S(O)₂NHT³;
NHT³; or
T³;
whereby when R⁸ is T¹-T² and represents -C₁₋₆ alkyl and T is T¹-T² and represents -C₁₋₆ alkyl then R⁸ and T may form together a 3 to 7 membered cyclic group containing one nitrogen atom;
R¹¹ is selected from the group consisting of
C₁₋₆ alkyl;
O-C₁₋₆ alkyl;
COO-C₁₋₆ alkyl;
OC(O)-C₁₋₆ alkyl;
C(O)N(R¹⁴)-C₁₋₆ alkyl;
S(O)₂N(R¹⁶)-C₁₋₆ alkyl;
S(O)-C₁₋₆ alkyl;
S(O)₂-C₁₋₆ alkyl; and
N(R¹⁷)S(O)₂-C₁₋₆ alkyl;
wherein each C₁₋₆ alkyl is optionally substituted with one, or independently from each other, more of F, COOR¹⁸, C(O)N(R¹⁹R²⁰), S(O)₂N(R²¹R²²), OR²³, N(R²⁴R²⁵), T³, O-T³ or N(R²⁶)-T³;
R¹² is selected from the group consisting of
C₁₋₆ alkyl;
O-C₁₋₆ alkyl;
N(R¹³)-C₁₋₆ alkyl;
COO-C₁₋₆ alkyl;
OC(O)-C₁₋₆ alkyl;
C(O)N(R¹⁴)-C₁₋₆ alkyl;
N(R¹⁵)-C(O)-C₁₋₆ alkyl;
S(O)₂N(R¹⁶)-C₁₋₆ alkyl;
S(O)-C₁₋₆ alkyl;
S(O)₂-C₁₋₆ alkyl; and
-N(R¹⁷)S(O)₂-C₁₋₆ alkyl;
wherein each C₁₋₆ alkyl is optionally substituted with one, or independently from each other, more of F, COOR¹⁸, C(O)N(R¹⁹R²⁰), S(O)₂N(R²¹R²²), OR²³, N(R²⁴R²⁵), T³, O-T³ or N(R²⁶)-T³;
R¹³, R¹⁴, R¹⁵, R¹⁶, R¹⁷, R¹⁸, R¹⁹, R²⁰, R²¹, R²², R²³, R²⁴ R²⁵ and R²⁶ are independently from each other H or C₁₋₆ alkyl;
T³ is selected from the group consisting of
phenyl;
naphthyl;
wherein phenyl and naphthyl are optionally substituted with one, or independently from each other, more of
halogen;
CN;
COOH;
OH;
C(O)NH₂;
S(O)₂NH₂;
C₁₋₆ alkyl;
O-C₁₋₆ alkyl;
COO-C₁₋₆ alkyl;
OC(O)-C₁₋₆ alkyl;
C(O)N(R²⁷)-C₁₋₆ alkyl;
S(O)₂N(R²⁸)-C₁₋₆ alkyl;
S(O)₂-C₁₋₆ alkyl; or
N(R²⁹)S(O)₂-C₁₋₆ alkyl;
heterocycle;
heterobicycle; and
C₃₋₇ cycloalkyl;
wherein C₃₋₇ cycloalkyl, heterocycle and heterobicycle are optionally substituted with one, or independently from each other, more of
halogen;
CN;
OH;
=O, where the ring is at least partially saturated;
NH₂;
COOH;
C(O)NH₂;
S(O)₂NH₂;
C₁₋₆ alkyl;
O-C₁₋₆ alkyl;
N(R³⁰)-C₁₋₆ alkyl;
COO-C₁₋₆ alkyl;
OC(O)-C₁₋₆ alkyl;
C(O)N(R³¹)-C₁₋₆ alkyl;
N(R³²)-C(O)-C₁₋₆ alkyl;
S(O)₂N(R³³)-C₁₋₆ alkyl;
S(O)₂-C₁₋₆ alkyl; or
-N(R³⁴)S(O)₂-C₁₋₆ alkyl;
and
R²⁷, R²⁸, R²⁹, R³⁰, R³¹, R³², R³³ and R³⁴ are independently from each other
H or C₁₋₆ alkyl.

2. A compound according to claim 1 of formula (la) 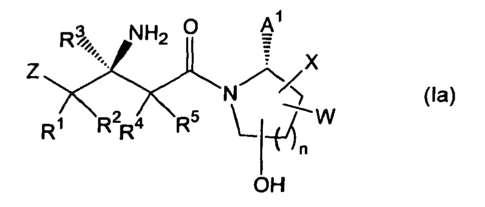 or a pharmaceutically acceptable salt thereof, wherein Z, R¹-R⁵, A¹, X, W and n have the meaning as indicated in claim 1.

3. A compound according to claim 1 or 2, wherein Z is phenyl or heterocycle and Z is optionally substituted independently from each other with up to 3 of CI, F, CN, CH₃ or OCH₃.

4. A compound according to any of claims 1 to 3, wherein R¹, R², R⁴, R⁵ are independently from each other selected from the group consisting of H, F, OH CH₃, OCH₃.

5. A compound according to any of claims 1 to 4, wherein R³ is H.

6. A compound according to any of claims 1 to 5, wherein n is 1.

7. A compound according to any of claims 1 to 6, wherein Y is -O-, -N(R⁸)- or -S(O)₂-.

8. A compound according to any of claims 1 to 7, wherein R⁸ is selected from the group consisting of H, CH₃, COOH, COOCH₃, C(O)NH₂, C(O)N(CH₃)₂, and S(O)₂CH₃.

9. A compound according to any of claims 1 to 8, wherein T is T¹-T² or T² and wherein T¹ is selected from the group consisting of
- CH₂-;
- C(O)-;
- C(O)-CH₂-;
- C(O)O-;
- C(O)O-CH₂-;
- C(O)NH-;
- C(O)NH-CH₂-,
- S(O)₂-; and
- S(O)₂-CH₂-.

10. A compound according to claim 9, wherein T is T¹-T² or T² and wherein T¹ is selected from the group consisting of -CH₂-; -C(O)-; -C(O)NH- and -S(O)₂-.

11. A compound according to any of claims 1 to 10, wherein A¹ is -CH₂-N(R⁸)-T, and wherein R⁸ is H or S(O)₂CH₃.

12. A compound according to any of claims 1 to 11, wherein T² is F, CF₃, C₁₋₆ alkyl optionally substituted with one or more halogen atoms, OCH₃, OH or C₃₋₅ cycloalkyl and
wherein C₃₋₅ cycloalkyl is optionally substituted with one, or independently from each other, more of halogen; OH; or OCH₃.

13. A compound according to claim 1 selected from the group consisting of 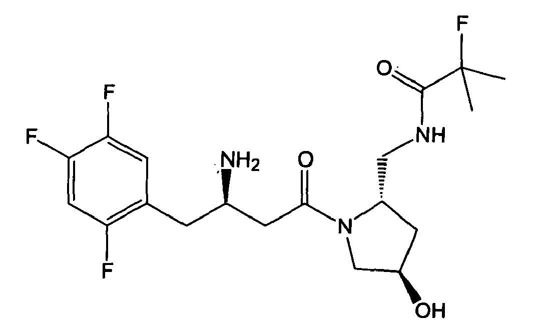 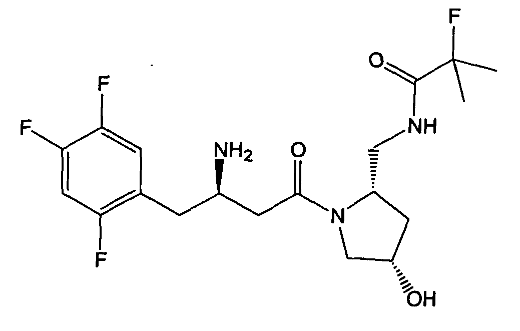 or a pharmaceutically acceptable salt thereof.

14. A compound of formula (II) 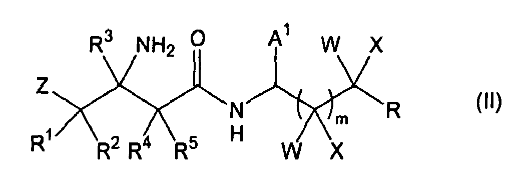 or a pharmaceutically acceptable salt thereof, wherein
Z is selected from the group consisting of
phenyl;
naphthyl;
C₃₋₇ cycloalkyl;
heterocycle; and
heterobicycte;
wherein Z is optionally substituted with one, or independently from each other, more of
halogen;
CN;
OH;
=O, where the ring is at least partially saturated;
C₁₋₆ alkyl, optionally substituted with one or more F; and
O-C₁₋₆ alkyl, optionally substituted with one or more F;
R¹, R², R⁴ and R⁵ are independently from each other selected from the group consisting of
H;
F;
OH;
C₁₋₆ alkyl, optionally substituted with one or more F; and
O-C₁₋₆ alkyl, optionally substituted with one or more F;
and/or R¹ and R² optionally form together C₃₋₇ cycloalkyl, which is optionally substituted with one or more F;
and/or R² and R³ optionally form together C₃₋₇ cycloalkyl, which is optionally substituted with one or more F;
and/or R³ and R⁴ optionally form together C₃₋₇ cycloalkyl, which is optionally substituted with one or more F;
and/or R⁴ and R⁵ optionally form together C₃₋₇ cycloalkyl, which is optionally substituted with one or more F;
R³ is H or C₁₋₆ alkyl;
R is
COOH or NR'R" and
wherein R' and R" are independently from each other selected from
H;
C₁₋₄ alkyl, optionally substituted with one or more F;
or R' and R" may form together a C₃₋₇ cycloalkyl, which is optionally substituted with one or more F;
X are independently from each other selected from
H;
F;
CH₃;
OCH₃;
OH;
W are independently from each other H or F;
m is 0, 1 or 2;
A¹ is -C(R⁶R⁷)-Y-T;
R⁶ and R⁷ are independently from each other selected from the group consisting of
H;
F; and
C₁₋₆ alkyl, optionally substituted with one or more F;
or R⁶ and R⁷ optionally form together C₃₋₇ cycloalkyl, which is optionally substituted with one or more F;
Y is selected from the group consisting of
-O-;
-C₁₋₆ alkyl-O-;
-N(R⁸)-;
-C₁₋₆ alkyl-N(R⁸)-;
-S-;
-C₁₋₆ alkyl-S-;
-S(O)-;
-C₁₋₆ alkyl-S(O)-;
-S(O)₂-; and
-C₁₋₆ alkyl-S(O)₂-;
wherein each C₁₋₆ alkyl is optionally substituted with one or more F;
R⁸ and T are independently from each other T¹-T² or T²;
T¹ is selected from the group consisting of
-C₁₋₆ alkyl-;
-C₁₋₆ alkyl-O-;
-C₁₋₆ alkyl-N(R⁹)-;
-C(O)-;
-C(O)-C₁₋₆ alkyl-;
-C(O)-C₁₋₆ alkyl-O-;
-C(O)-C₁₋₆ alkyl-N(R⁹)-;
-C(O)O-;
-C(O)O-C₁₋₆ alkyl-;
-C(O)O-C₁₋₆ alkyl-O-;
-C(O)O-C₁₋₆ alkyl-N(R⁹)-;
-C(O)N(R⁹)-;
-C(O)N(R⁹)-C₁₋₆ alkyl-;
-C(O)N(R⁹)-C₁₋₆ alkyl-O-;
-C(O)N(R⁹)-C₁₋₆ alkyl-N(R¹⁰)-;
-S(O)₂-;
-S(O)₂-C₁₋₆ alkyl-;
-S(O)₂-C₁₋₆ alkyl-O-; and
-S(O)₂-C₁₋₆ alkyl-N(R⁹)-;
wherein each C₁₋₆ alkyl is optionally substituted with one or more F;
R⁹ and R¹⁰ are independently from each other H or C₁₋₆ alkyl, optionally substituted with one or more F;
T² is selected from the group consisting of
H;
CF₃;
phenyl;
naphthyl;
wherein phenyl and naphthyl are optionally substituted with one, or independently from each other, more of
halogen;
CN;
R¹¹;
COOH;
OH;
C(O)NH₂;
S(O)₂NH₂;
COOT³;
OT³;
C(O)NHT³;
S(O)₂NHT³; or
T³;
C₃₋₇ cycloalkyl;
heterocycle; and
heterobicycle;
wherein C₃₋₇ cycloalkyl, heterocycle and heterobicycle are optionally substituted with one, or independently from each other, more of
halogen;
CN;
R¹²;
OH;
=O, where the ring is at least partially saturated;
NH₂
COOH;
C(O)NH₂;
S(O)₂NH₂;
COOT³;
OT³;
C(O)NHT³;
S(O)₂NHT³;
NHT³; or
T³;
whereby when R⁸ is T¹- T² and represents -C₁₋₆ alkyl and T is T¹- T² and represents -C₁₋₆ alkyl then R⁸ and T may form together a 3 to 7 membered cyclic group containing one nitrogen atom;
R¹¹ is selected from the group consisting of
C₁₋₆ alkyl;
O-C₁₋₆ alkyl;
COO-C₁₋₆ alkyl;
OC(O)-C₁₋₆ alkyl;
C(O)N(R¹⁴)-C₁₋₆ alkyl;
S(O)₂N(R¹⁶)-C₁₋₆ alkyl;
S(O)-C₁₋₆ alkyl;
S(O)₂-C₁₋₆ alkyl; and
N(R¹⁷)S(O)₂-C₁₋₆ alkyl;
wherein each C₁₋₆ alkyl is optionally substituted with one, or independently from each other, more of F, COOR¹⁸, C(O)N(R¹⁹R²⁰), S(O)₂N(R²¹R²²), OR²³, N(R²⁴R²⁵), T³, O-T³ or N(R²⁶)-T³;
R¹² is selected from the group consisting of
C₁₋₆ alkyl;
O-C₁₋₆ alkyl;
N(R¹³)-C₁₋₆ alkyl;
COO-C₁₋₆ alkyl;
OC(O)-C₁₋₆ alkyl;
C(O)N(R¹⁴)-C₁₋₆ alkyl;
N(R¹⁵)-C(O)-C₁₋₆ alkyl;
S(O)₂N(R¹⁶)-C₁₋₆ alkyl;
S(O)-C₁₋₆ alkyl;
S(O)₂-C₁₋₆ alkyl; and
-N(R¹⁷)S(O)₂-C₁₋₆ alkyl;
wherein each C₁₋₆ alkyl is optionally substituted with one, or independently from each other, more of F, COOR¹⁸, C(O)N(R¹⁹R²⁰), S(O)₂N(R²¹R²²), OR²³, N(R²⁴R²⁵), T³, O-T³ or N(R²⁶)-T³;
R¹³ R¹⁴, R¹⁵, R¹⁶, R¹⁷, R¹⁸, R¹⁹, R²⁰, R²¹, R²², R²³, R²⁴, R²⁵ and R²⁶ are independently from each other H or C₁₋₆ alkyl;
T³ is selected from the group consisting of
phenyl;
naphthyl;
wherein phenyl and naphthyl are optionally substituted with one, or independently from each other, more of
halogen;
CN;
COOH;
OH;
C(O)NH₂;
S(O)₂NH₂;
C₁₋₆ alkyl;
O-C₁₋₆ alkyl;
COO-C₁₋₆ alkyl;
OC(0)-C₁₋₆ alkyl;
C(O)N(R²⁷)-C₁₋₆ alkyl;
S(O)₂N(R²⁸)-C₁₋₆ alkyl;
S(O)₂-C₁₋₆ alkyl; or
N(R²⁹)S(O)₂-C₁₋₆ alkyl;
heterocycle;
heterobicycle; and
C₃₋₇ cycloalkyl;
wherein C₃₋₇ cycloalkyl, heterocycle and heterobicycle are optionally substituted with one, or independently from each other, more of
halogen;
CN;
OH;
=O, where the ring is at least partially saturated;
NH₂;
COOH;
C(O)NH₂;
S(O)₂NH₂;
C₁₋₆ alkyl;
O-C₁₋₆ alkyl;
N(R³⁰)-C₁₋₆ alkyl;
COO-C₁₋₆ alkyl;
OC(O)-C₁₋₆ alkyl;
C(O)N(R³¹)-C₁₋₆ alkyl;
N(R³²)-C(O)-C₁₋₆ alkyl;
S(O)₂N(R³³)-C₁₋₆ alkyl;
S(O)₂-C₁₋₆ alkyl; or
-N(R³⁴)S(O)₂-C₁₋₆ alkyl;
and
R²⁷, R²⁸, R²⁹, R³⁰, R³¹, R³², R³³ and R³⁴ are independently from each other H or C₁₋₆ alkyl.

15. A compound according to claim 14 of formula (IIa) 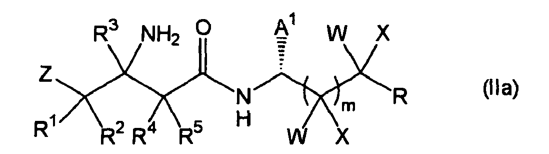 or a pharmaceutically acceptable salt thereof, wherein Z, R¹-R⁵, A¹, X, W, R and m have the meaning as indicated in claim 14.

16. A compound according to claim 14 or 15, wherein Z is phenyl or heterocycle and Z is optionally substituted independently from each other with up to 3 of Cl, F, CN, CH₃ or OCH₃.

17. A compound according to any of claims 14 to 16, wherein R¹, R², R⁴ and R⁵ are independently from each other selected from the group consisting of H, F, OH, CH₃, and OCH₃.

18. A compound according to any of claims 14 to 17, wherein R³ is H.

19. A compound according to any of claims 14 to 18, wherein m is 1 or 2.

20. A compound according to any of claims 14 to 19, wherein Y is -O-, -N(R⁸)- or -S(O)₂-.

21. A compound according to any of claims 14 to 20, wherein R⁸ is selected from the group consisting of H, CH₃, COOH, COOCH₃, C(O)NH₂, C(O)N(CH₃)₂, and S(O)₂CH₃.

22. A compound according to any of claims 14 to 21, wherein T is T¹-T² or T² and wherein T¹ is selected from the group consisting of
- CH₂-;
- C(O)-;
- C(O)-CH₂-;
- C(O)O-;
- C(O)O-CH₂-;
- C(O)NH-;
- C(O)NH-CH₂-;
- S(O)₂-; and
- S(O)₂-CH₂-.

23. A compound according to claim 22, wherein T is T¹-T² or T² and wherein T¹ is selected from the group consisting of -CH₂-; -C(O)-; -C(O)NH- and -S(O)₂-.

24. A compound according to any of claims 14 to 23, wherein A¹ is -CH₂-N(R⁸)-T, and wherein R⁸ is H or S(O)₂CH₃.

25. A compound according to any of claims 14 to 24, wherein T² is F, CF₃, C₁₋₆ alkyl optionally substituted with one or more halogen atoms, C₃₋₅ cycloalkyl
wherein C₃₋₅ cycloalkyl is optionally substituted with one, or independently from each other, more of
halogen;
OH;
OCH₃.

26. A prodrug compound of a compound according to any one of claims 1 to 25.

27. A pharmaceutical composition comprising at least one compound or pharmaceutically acceptable salt thereof according to any one of claims 1 to 26 together with a pharmaceutically acceptable carrier.

28. A pharmaceutical composition according to claim 27, comprising one or more additional compounds or pharmaceutically acceptable salts thereof selected from the group consisting of another DPP-IV inhibitor; insulin sensitizers; PPAR agonists; biguanides; protein tyrosinephosphatase-IB (PTP-1 B) inhibitors; insulin and insulin mimetics; sulfonylureas and other insulin secretagogues; a-glucosidase inhibitors; glucagon receptor antagonists; GLP-1, GLP-1 mimetics, and GLP-1 receptor agonists; GIP, GIP mimetics, and GIP receptor agonists; PACAP, PACAP mimetics, and PACAP receptor 3 agonists; cholesterol lowering agents; HMG-CoA reductase inhibitors; sequestrants; nicotinyl alcohol; nicotinic acid or a salt thereof; PPARa agonists; PPARoly dual agonists; inhibitors of cholesterol absorption; acyl CoA : cholesterol acyltransferase inhibitors; anti-oxidants; PPARo agonists; antiobesity compounds; an ileal bile acid transporter inhibitor; and anti-inflammatory agents.

29. A compound or a pharmaceutically acceptable salt thereof of any one of claims 1 to 26 for use as a medicament.

30. Use of a compound or a pharmaceutically acceptable salt thereof of any of claims 1 to 26 for the manufacture of a medicament for the treatment or prophylaxis of non-insulin dependent (Type II) diabetes mellitus; hyperglycemia; obesity; insulin resistance; lipid disorders; dyslipidemia; hyperlipidemia; hypertriglyceridemia; hypercholestrerolemia; low HDL; high LDL; atherosclerosis; growth hormone deficiency; diseases related to the immune response; HIV infection; neutropenia; neuronal disorders; anxiety; depression; tumor metastasis; benign prostatic hypertrophy; gingivitis; hypertension; osteoporosis; diseases related to sperm motility; low glucose tolerance; insulin resistance; ist sequelae; vascular restenosis; irritable bowel syndrome; inflammatory bowel disease; including Crohn's disease and ulcerative colitis; other inflammatory conditions; pancreatitis; abdominal obesity; neurodegenerative disease; retinopathy; nephropathy; neuropathy; Syndrome X; ovarian hyperandrogenism (polycystic ovarian syndrome; Type n diabetes; or growth hormone deficiency.
